(19) Europäisches Patentamt
European Patent Office
Office européen des brevets

(11) Veröffentlichungsnummer: **0 507 188 A1**

(12) **EUROPÄISCHE PATENTANMELDUNG**

(21) Anmeldenummer: **92105039.9**

(22) Anmeldetag: **24.03.92**

(51) Int. Cl.5: **C07H 19/06**, C07H 19/10, C07D 405/04, C07D 405/14, A61K 31/70, A61K 31/505

(30) Priorität: **05.04.91 DE 4110977**

(43) Veröffentlichungstag der Anmeldung: **07.10.92 Patentblatt 92/41**

(84) Benannte Vertragsstaaten: **AT BE CH DE DK ES FR GB GR IT LI LU NL PT SE**

(71) Anmelder: **BAYER AG**

**W-5090 Leverkusen 1 Bayerwerk(DE)**

(72) Erfinder: **Häbich, Dieter, Dr.**
**Krummacherstrasse 82**
**W-5600 Wuppertal(DE)**
Erfinder: **Neumann, Rainer, Dr.**
**Albert-Einstein-Strasse 27**
**W-5024 Pulheim 2(DE)**

(54) **Substituierte 2',3'-Didesoxy-5-trifluormethyluridine, Verfahren zu ihrer Herstellung und ihre Verwendung in Arzneimitteln.**

(57) Die Erfindung betrifft 2',3'-Didesoxy-5-trifluormethyluridine der allgemeinen Formel I

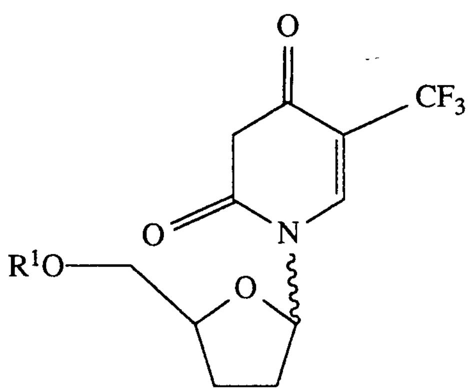

(I)

worin $R^1$ die in der Beschreibung angegebene Bedeutung hat, Verfahren zu ihrer Herstellung sowie ihre Verwendung als Arzneimittel, insbesondere als Arzneimittel gegen Hepatitis.

EP 0 507 188 A1

Die Erfindung betrifft 2',3'-Didesoxy-5-trifluormethyluridine, Verfahren zu ihrer Herstellung sowie ihre Verwendung als Arzneimittel, insbesondere als Arzneimittel gegen Hepatitis.

Weltweit sind ca. 300 Millionen Menschen an Hepatitis B erkrankt, wobei allein in den USA und Europa ca. 1 Millionen Neuerkrankungen pro Jahr registriert werden.

Es ist bekannt, daß bestimmte, in 2'- und 3'-Stellung unsubstituierte 2',3'-Didesoxynucleoside gegen Hepatitis-(B)-Viren wirken [vgl. U.S. Pat. Appl. 351 502 (15.8.89); EP 302 760]. In der WO 90/140 79 wird explizit 2',3'-Didesoxy-cytidin (DDC) als Arzneimittel gegen Hepatitis B beschrieben. Außerdem ist durch die Publikation [D.L.J. Tyrell et al., Biochem. Biophys. Res. Commun. 156, 1144 - 1148 (1988)] bekannt, daß nur 2',3'-Didesoxypurin-, nicht aber 2',3'-Didesoxypyrimidinnucleoside, eine Wirksamkeit gegen DHBV (duck hepatitis B virus) zeigen. Es wird außerdem darauf hingewiesen, daß in der EP 217 580 die für das dortige Verfahren einzig richtige Ausgangsverbindung 2'-Didesoxy-5-trifluormethyluridin irrtümlicherweise unter der falschen Nomenklatur - 2',3'-Didesoxy-5-trifluormethyluridin - publiziert (Beispiel 34) und somit auch von Chemical Abstracts unter RN-Nummer 108 441/81/2 als Struktur aufgenommen wurde, ohne daß diese Verbindung bisher von dem Bedeutungsumfang einer publizierten Anmeldung oder eines Patentes erfaßt oder als konkreter Stoffvertreter aufgeführt wurde.

Die Erfindung betrifft 2',3'-Didesoxy-5-trifluormethyluridine der allgemeinen Formel (I)

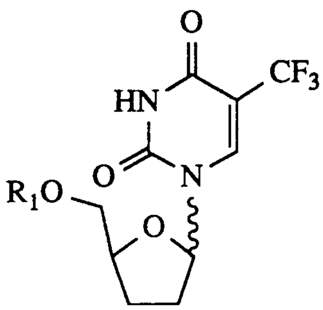 (I),

worin

$R^1$ für Wasserstoff oder eine Hydroxyschutzgruppe steht,
für 4-Methylbenzoyl oder geradkettiges oder verzweigtes Acyl mit bis zu 18 Kohlenstoffatomen steht, das gegebenenfalls durch Carboxy substituiert ist,
für eine Aminosäure steht, die gegebenenfalls durch eine in der Peptidchemie übliche Aminoschutzgruppe substituiert ist,
für einen in vivo spaltbaren Esterrest steht, oder
für einen Rest der Formeln

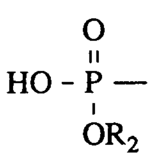 , 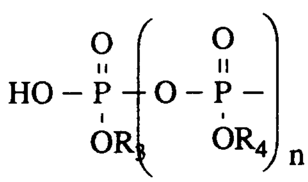 , 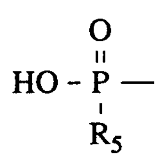

steht, worin

$R^2$, $R^3$ und $R^4$ gleich oder verschieden sind und Wasserstoff oder ein Alkali- oder Erdalkalimetallatom bedeuten,

n die Zahl 1 oder 2 bedeutet,

$R^5$ geradkettiges oder verzweigtes Alkyl mit bis zu 6 Kohlenstoffatomen bedeutet, das gegebenenfalls durch Halogen substituiert ist

und deren physiologisch unbedenklichen Salze.

Im Rahmen der vorliegenden Erfindung werden physiologisch unbedenkliche Salze bevorzugt. Physiologisch unbedenkliche Salze der erfindungsgemäßen Verbindungen können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure,

Milchsäure, Weinsäure, Zitronensäure, Fumarsäure, Maleinsäure oder Benzoesäure.

Als Salze können auch Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze abgeleitet vom Ammoniak oder organischen Aminen wie beispielsweise Diethylamin, Triethylamin, Ethyldiisopropylamin, Prokain, N-Methylmorpholin oder N-Methylpiperidin oder Dihydroabietylamin.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

a) 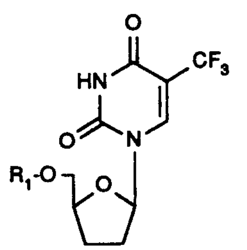

D, ß oder (S, R)

b) 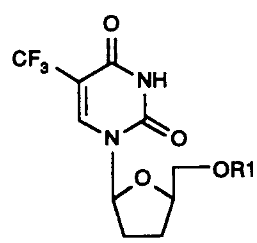

L, ß oder (R, S)

c) 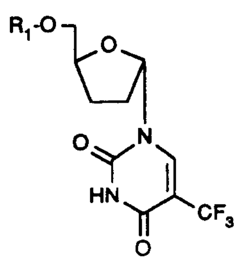

D, α oder (S, S)

d) 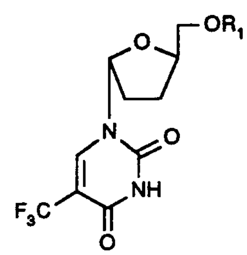

L, α oder (R, R)

Aminoschutzgruppen im Rahmen der Erfindung sind die üblichen in der Peptid-Chemie verwendeten Aminoschutzgruppen.

Hierzu gehören bevorzugt: Benzyloxycarbonyl, 3,4-Dimethoxybenzyloxycarbonyl, 3,5-Dimethoxybenzyloxycarbonyl, 2,4-Dimethoxybenzyloxycarbonyl, 4-Methoxybenzyloxycarbonyl, 4-Nitrobenzyloxycarbonyl, 2-Nitrobenzyloxycarbonyl, 2-Nitro-4,5-dimethoxybenzyloxycarbonyl, Methoxycarbonyl, Ethoxycarbonyl, Propoxycarbonyl, Isopropoxycarbonyl, Butoxycarbonyl, Isobutoxycarbonyl, tert.Butoxycarbonyl, Allyloxycarbonyl, Vinyloxycarbonyl, 2-Nitrobenzyloxycarbonyl, 3,4,5-Trimethoxybenzyloxycarbonyl, Cyclohexylcarbonyl, 1,1-Dimethylethoxycarbonyl, Adamantylcarbonyl, Phthaloyl, 2,2,2-Trichlorethoxycarbonyl, 2,2,2-Trichlor-tertbutoxycarbonyl, Menthyloxycarbonyl, Phenoxycarbonyl, 4-Nitrophenoxycarbonyl, Fluorenyl-9-methoxycarbonyl, Formyl, Acetyl, Propionyl, Pivaloyl, 2-Chloracetyl, 2-Bromacetyl, 2,2,2-Trifluoracetyl, 2,2,2-Trichloracetyl, Benzoyl, 4-Chlorbenzoyl, 4-Brombenzoyl, 4-Nitrobenzoyl, Phthalimido, Isovaleroyl oder Benzyloxymethylen, 4-Nitrobenzyl, 2,4-Dinitrobenzyl, 4-Nitrophenyl.

Aminosäuren (-NH-CHR-CO-) im Rahmen der oben angegebenen Definition ($R^1$) stehen im allgemeinen für die natürlichen Aminosäuren aus der Reihe Alanin, Asparagin, Asparaginsäure, Glutaminsäure, Glutamin, Histidin, Leucin, Methionin, Prolin, Threonin, Tyrosin, Arginin, Cystein, Glycin, Isoleucin, Lysin, Phenylalanin, Serin, Tryptophan oder Valin, wobei die Verknüpfung zum Sauerstoff ($R^1$-O-) in der allgemeinen Formel (I) über die Carbonylgruppe erfolgt, während die endständige Aminogruppe entweder als freie $NH_2$-Gruppe oder gegebenenfalls durch eine der oben aufgeführten Schutzgruppen substituiert vorliegt.

Hydroxyschutzgruppe im Rahmen der oben angegebenen Definition steht im allgemeinen für eine Schutzgruppe aus der Reihe: Trimethylsilyl, Triethylsilyl, Triisopropylsilyl, tert.Butyl-dimethylsilyl, tert.-Butyldiphenylsilyl, Triphenylsilyl, Trimethylsilylethoxycarbonyl, Benzyl, Triphenylmethyl (Trityl), Monomethoxytrityl (MMTr), Dimethoxytrityl (DMTr), Benzyloxycarbonyl, 2-Nitrobenzyl, 4-Nitrobenzyl, 2-Nitrobenzyloxycarbonyl, 4-Nitrobenzyloxycarbonyl, tert. Butyloxycarbonyl, Allyloxycarbonyl, 4-Methoxybenzyl, 4-Methoxybenzyloxycarbonyl, Formyl, Acetyl, Trichloracetyl, 2,2,2-Trichlorethoxycarbonyl, 2,4-Dimethoxybenzyl, 2,4-Dimethoxybenzyloxycarbonyl, Methoxymethyl, Methylthiomethyl, Methoxyethoxymethyl, [2-(Trimethylsilyl)ethoxy]methyl, 2-(Methylthiomethoxy)ethoxycarbonyl, Tetrahydropyranyl, Benzoyl, 4-Methylbenzoyl, 4-Nitrobenzoyl, 4-Fluorbenzoyl, 4-Chlorbenzoyl oder 4-Methoxybenzoyl.

In vivo leicht abspaltbare Esterreste stehen im Rahmen der oben angebenen Definition $R^1$ für pharmazeutisch verträgliche Esterreste, die in vivo unter Freisetzung der OH-Gruppe (R = H) leicht abgespalten werden können. Beispiele für solche Gruppen sind aus der EP 357 495 bekannt.

Bevorzugte in vivo abspaltbare Estergruppen sind die der folgenden Formeln:

$$(CH_3)_3C\text{-}CO\text{-}O\text{-}CH_2\text{-} \quad , \quad CH_3\text{-}CO\text{-}O\text{-}\underset{|}{CH}\text{-}CH_3 \quad ,$$

$$CH_3\text{-}CH_2O\text{-}CO\text{-}O\text{-}\underset{|}{CH}\text{-}CH_3$$

In Abhängigkeit von der jeweiligen Bedeutung des Restes $R^1$ können die erfindungsgemäßen Verbindungen der allgemeinen Formel (I) als Prodrugs fungieren, d.h. als pharmazeutisch verträgliche Verbindungen, die bei oder nach Applikation, gegebenenfalls durch ein verbessertes pharmakologisches und pharmakokinetisches Verhalten, weitere erfindungsgemäß anwendbare Verbindungen der allgemeinen Formel (I) in welcher z.B. $R^1$ = H ist, als Stoffwechselprodukte oder Abbauprodukte bereitstellen [vgl. dazu J. Pharm. Sci. 79, 531 (1990); EP 357 495; EP 362 967; EP 366 386 und WP 90/049 69].

Bevorzugt sind Verbindungen der allgemeinen Formel (I),
in welcher

$R^1$ für Wasserstoff, tert.Butyldimethylsilyl, Trimethylsilyl, Triphenylmethyl, Benzoyl, 4-Methylbenzoyl oder Acetyl steht, oder
für geradkettiges oder verzweigtes Acyl mit bis zu 16 Kohlenstoffatomen steht, das gegebenenfalls durch Carboxy substituiert ist, oder
für Leucin (Leu), Valin (Val), Isoleucin (Ile), Phenylalanin (Phe), Asparaginsäure (Asp) oder Glutaminsäure (Glu) steht, die gegebenenfalls an der NH-Funktion durch tert.Butoxycarbonyl (Boc) oder Benzyloxycarbonyl (Z) substituiert sind, oder
für einen in vivo leicht spaltbaren Esterrest der Formeln

$$(CH_3)_3C\text{-}CO\text{-}O\text{-}CH_2\text{-} \quad , \quad CH_3\text{-}CO\text{-}O\text{-}\underset{|}{CH}\text{-}CH_3 \quad ,$$

$$CH_3\text{-}CH_2O\text{-}CO\text{-}O\text{-}\underset{|}{CH}\text{-}CH_3$$

steht, oder für einen Rest der Formeln

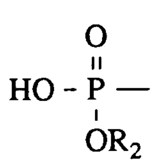 , 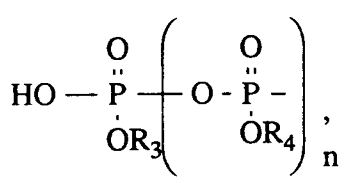 , 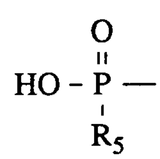

steht, worin

$R^2$, $R^3$ und $R^4$ gleich oder verschieden sind und Wasserstoff oder ein Natriummetallatom bedeuten,
n die Zahl 1 oder 2 bedeutet,

$R^5$ geradkettiges oder verzweigtes Alkyl mit bis zu 4 Kohlenstoffatomen bedeutet, das gegebenenfalls durch Chlor substituiert ist

und deren physiologisch unbedenklichen Salze.

Besonders bevorzugt sind Verbindungen der allgemeinen Formel (I), in welcher

$R^1$ für Wasserstoff, tert.Butyldimethylsilyl, 4-Methylbenzoyl oder Benzoyl steht, oder für geradkettiges oder verzweigtes Acyl mit bis zu 14 Kohlenstoffatomen steht, das gegebenenfalls durch Carboxy substituiert ist, oder für Leucin (Leu) oder Asparaginsäure (Asp) steht, die gegebenenfalls an der -NH-Funktion durch die Schutzgruppe Boc substituiert sind, oder für einen in vivo leicht spaltbaren Esterrest der Formel

$(CH_3)_3C\text{-}CO\text{-}O\text{-}CH_2\text{-}$ , $CH_3\text{-}CO\text{-}O\text{-}CH(\text{-})\text{-}CH_3$ ,

$CH_3\text{-}CH_2O\text{-}CO\text{-}O\text{-}CH(\text{-})\text{-}CH_3$

steht, oder

für einen Rest der Formeln

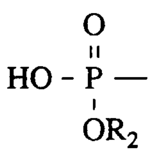 , 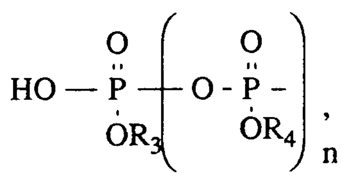 , 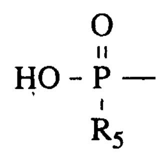

steht, worin

$R^2$, $R^3$ und $R^4$ gleich oder verschieden sind und Wasserstoff oder ein Natriummetallatom bedeuten,

n die Zahl 1 oder 2 bedeutet,

$R^5$ Methyl, Ethyl oder Chlormethyl bedeutet

und deren physiologisch unbedenklichen Salze.

Außerdem wurden Verfahren zur Herstellung der erfindungsgemäßen Verbindungen der allgemeinen Formel (I) gefunden, dadurch gekennzeichnet, daß man

[A] entweder 5-Trifluormethyluracil der Formel (II)

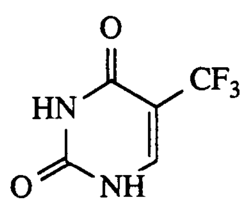 (II)

oder

Verbindungen der allgemeinen Formel (III)

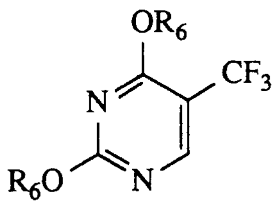

(III)

in welcher

$R^6$ für eine der oben aufgeführen Silylhydroxyschutzgruppen, vorzugsweise für Trimethylsilyl steht,

zunächst mit Verbindungen der allgemeinen Formel (IV)

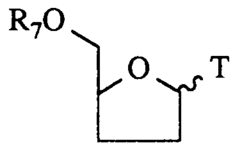

(IV),

in welcher

$R^7$ für eine der oben angegebenen Hydroxyschutzgruppen steht,

T für eine typische Abgangsgruppe wie Acetoxy, Brom, Chlor, Jod, Azido, 4-Nitrophenoxy, Brosylat, Tosylat, Mesylat, Trifluormethansulfonat oder Fluorsulfonat, vorzugsweise für Brom, Chlor oder Acetoxy steht,

in inerten Lösemitteln, gegebenenfalls in Anwesenheit einer Base und/oder eines Hilfsstoffes zu Verbindungen der allgemeinen Formel (Ia)

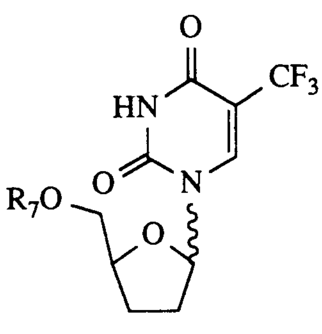

(Ia),

in welcher

$R^7$ die oben angegebene Bedeutung hat,

und die entweder als $\alpha,\beta$-Isomerengemisch oder als $\alpha$ und $\beta$-Isomer vorliegen,

umsetzt,

oder

[B] entweder die Verbindungen der allgemeinen Formel (III) mit den Verbindungen der allgemeinen Formel (IVa)

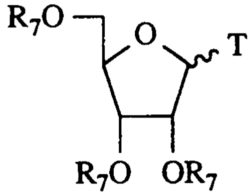

(IVa),

in welcher

$R^7$ und T die oben angegebene Bedeutung haben,
oder
1-(β-D-Ribofuranosyl)-5-trifluormethyl-uracil der Formel (V)

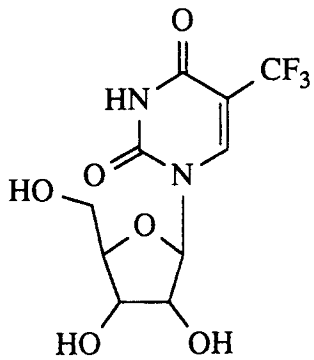

(V)

mit Verbindungen der allgemeinen Formel (VI)

$R^7$-W (VI),

in welcher
$R^7$ die oben angegebene Bedeutung hat
und
W die oben angegebene Bedeutung von T hat und mit dieser gleich oder verschieden ist,
in inerten Lösemitteln, gegebenenfalls in Anwesenheit einer Base und/oder eines Hilfsstoffes, und im Fall der Verbindung der Formel (V) unter gleichzeitiger Blockierung der vic-Hydroxyfunktionen und/oder der primären Hydroxyfunktion, und anschließender Freisetzung der vic-Hydroxyfunktionen zu den Verbindungen der allgemeinen Formel (VII)

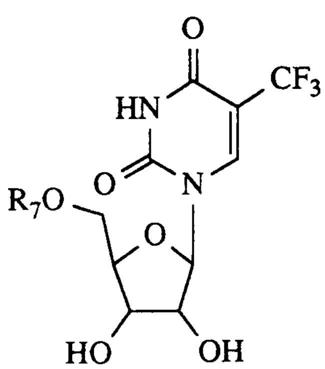

(VII),

in welcher
$R^7$ die oben angegebene Bedeutung hat,
umsetzt,
und in einem nächsten Schritt durch Umsetzung mit 1,1'-Thiocarbonyldiimidazol oder Orthoameisensäuredimethylester in die entsprechenden 1,4-Acetale bzw. 1,3-Thiocarbonaten der allgemeinen Formel (VIII)

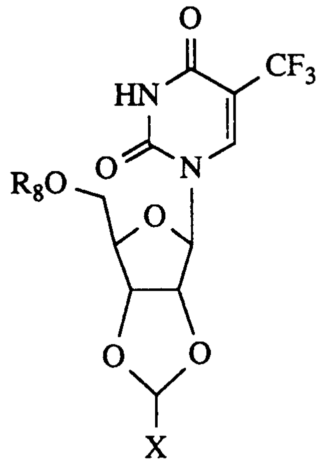

(VIII),

in welcher

$R^8$ die oben angegebene Bedeutung von $R^7$ hat oder für Wasserstoff steht

und

X für einen Rest der Formel =S oder $-OCH_3$ steht,

in inerten Lösemitteln, in Anwesenheit von Reduktionsmitteln und gegebenenfalls eines Kalalysators überführt,

und im Fall, daß $R^8$ für Wasserstoff steht, in einem Schritt durch Umsetzung mit Essigsäureanhydrid zunächst die primäre Hydroxyfunktion blockiert, den Acetalrest unter Ausbildung einer Doppelbindung abspaltet und anschließend diese nach üblichen Methoden hydriert,

und im Fall, daß X für den Rest der Formel =S steht, anschließend mit Trialkylphosphiten, wie Trimethyl-, -ethyl- oder -propylphosphit, vorzugsweise mit Triethylphosphit umsetzt,

und im Fall der Abspaltung des 1,3-Thiocarbonatrestes zunächst in Anwesenheit von Reduktionsmitteln und eines Katalysators reduziert, die Umsetzung mit 1,1'-Thiocarbonyldiimidazol wiederholt und zuletzt erneut reduziert,

oder

[C] Verbindungen der allgemeinen Formel (IX)

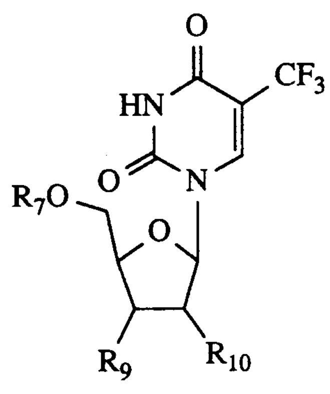

(IX),

in welcher

$R^7$ die oben angegebene Bedeutung hat,

und

$R^9$ und $R^{10}$ verschieden sind und für Wasserstoff oder Hydroxy stehen,

zunächst mit 1,1-Thiocarbonyldiimidazol oder Phenylthiocarbonylchlorid nach der unter [B] beschriebenen Methode zu den entsprechenden Thiocarbonylverbindungen der allgemeinen Formel (X)

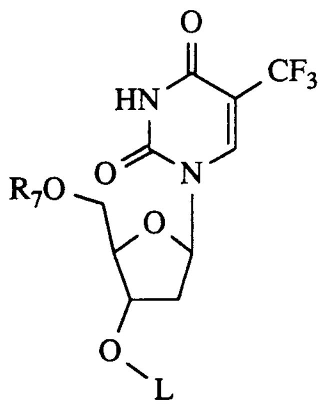

(X),

in welcher
$R^7$ die oben angegebene Bedeutung hat,
L für den Rest

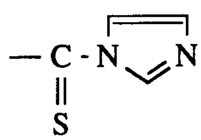

oder

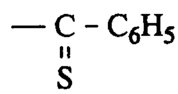

steht,
umsetzt,
und
anschließend wie unter [B] beschrieben die blockierte Hydroxygruppe eliminiert und die primäre Hydroxyfunktion deblockiert,
oder
im Fall, daß $R^1$ nicht für Wasserstoff oder für ein Phosphorsäurederivat steht,
[D] $\alpha,\beta$-2',3'-Didesoxy-5-trifluoruridin der Formel (Ib)

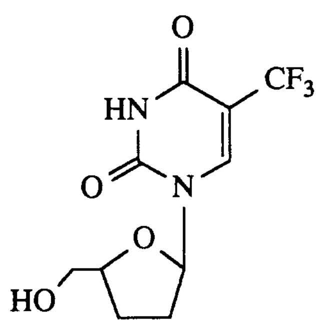

(Ib)

mit Verbindungen der allgemeinen Formel (XI),

$R^{11}$-Y (XI)

in welcher
$R^{11}$ die oben angegebene Bedeutung von $R^1$ hat aber nicht für Wasserstoff oder für ein Phosphorsäurederivat steht
und
Y die oben angegebene Bedeutung von T hat und mit dieser gleich oder verschieden ist,
in inerten Lösemitteln, gegebenenfalls in Anwesenheit einer Base und/oder eines Hilfsstoffes, umsetzt,
oder im Fall, daß $R^1$ für einen der oben aufgeführten Phosphorsäurereste steht,
die Verbindungen der allgemeinen Formel (Ib) zunächst mit Phosphortrichlorid, Trisimidazolylphosphin

oder Phosphoroxychlorid und anschließend gegebenenfalls mit Tributylammoniumdiphosphat oder mit Verbindungen der allgemeinen Formel (XII)

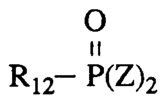 (XII),

worin

$R^{12}$ für einen Rest der Formeln

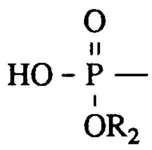 , 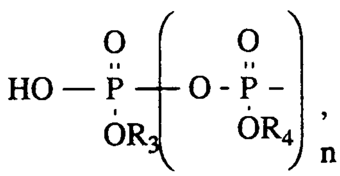 , 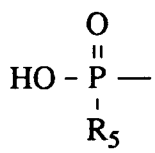

steht,

worin

$R^2$, $R^3$, $R^4$, $R^5$ und n die oben angebene Bedeutung haben,

und

Z für Halogen, vorzugsweise für Chlor steht,

in inerten Lösemitteln, gegebenenfalls in Anwesenheit einer Base und eines Alkalihalogenids, wie beispielsweise Natriumjodid nach üblicher Methode phosphoryliert,

und im Fall der freien Hydroxymethylfunktion ($R^1$ = H) die jeweilige Schutzgruppe nach üblicher Methode abspaltet und/oder gegebenenfalls eine chromatographische Trennung durchführt.

Die erfindungsgemäßen Verfahren können durch folgendes Formelschema beispielhaft erläutert werden:

[A]

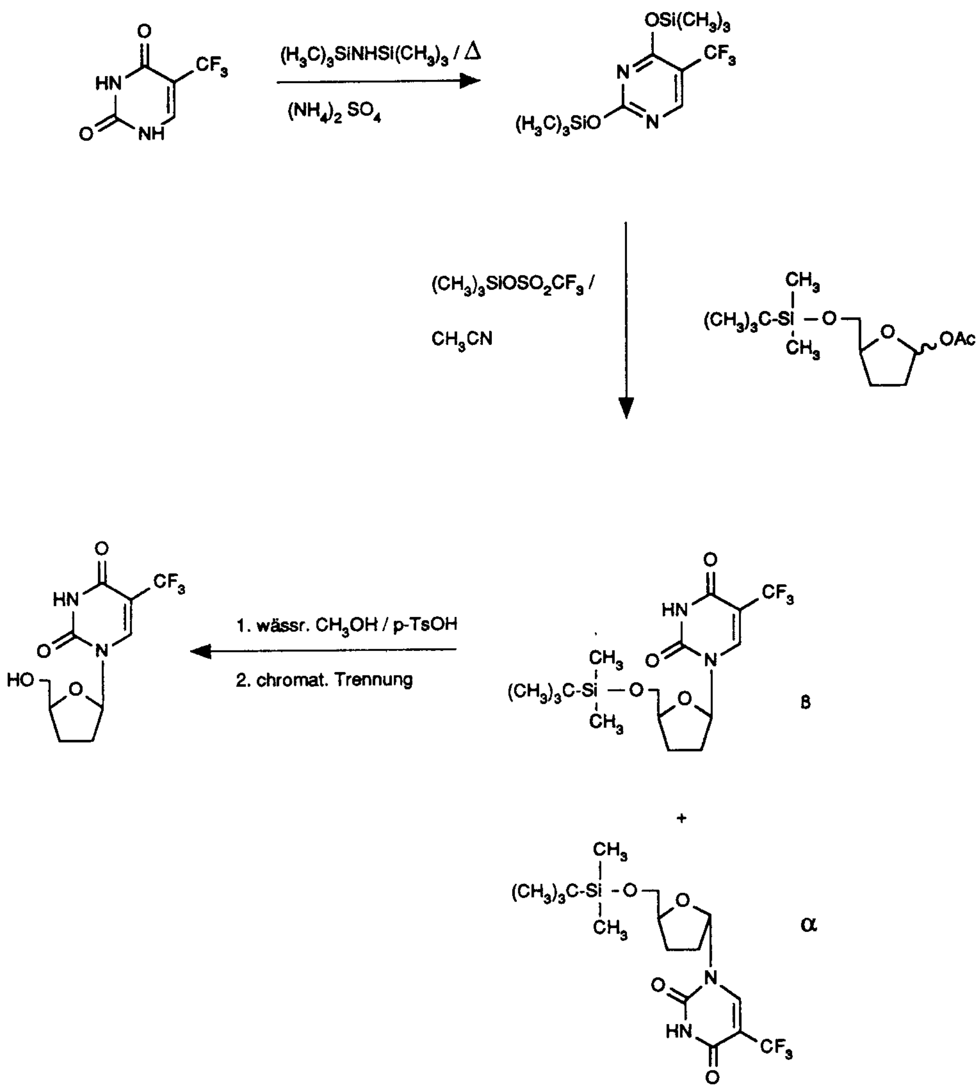
$(H_3C)_3SiNHSi(CH_3)_3$ / Δ
$(NH_4)_2$ $SO_4$
$(CH_3)_3SiOSO_2CF_3$ /
$CH_3CN$
1. wässr. $CH_3OH$ / p-TsOH
2. chromat. Trennung
β
+
α

[A]

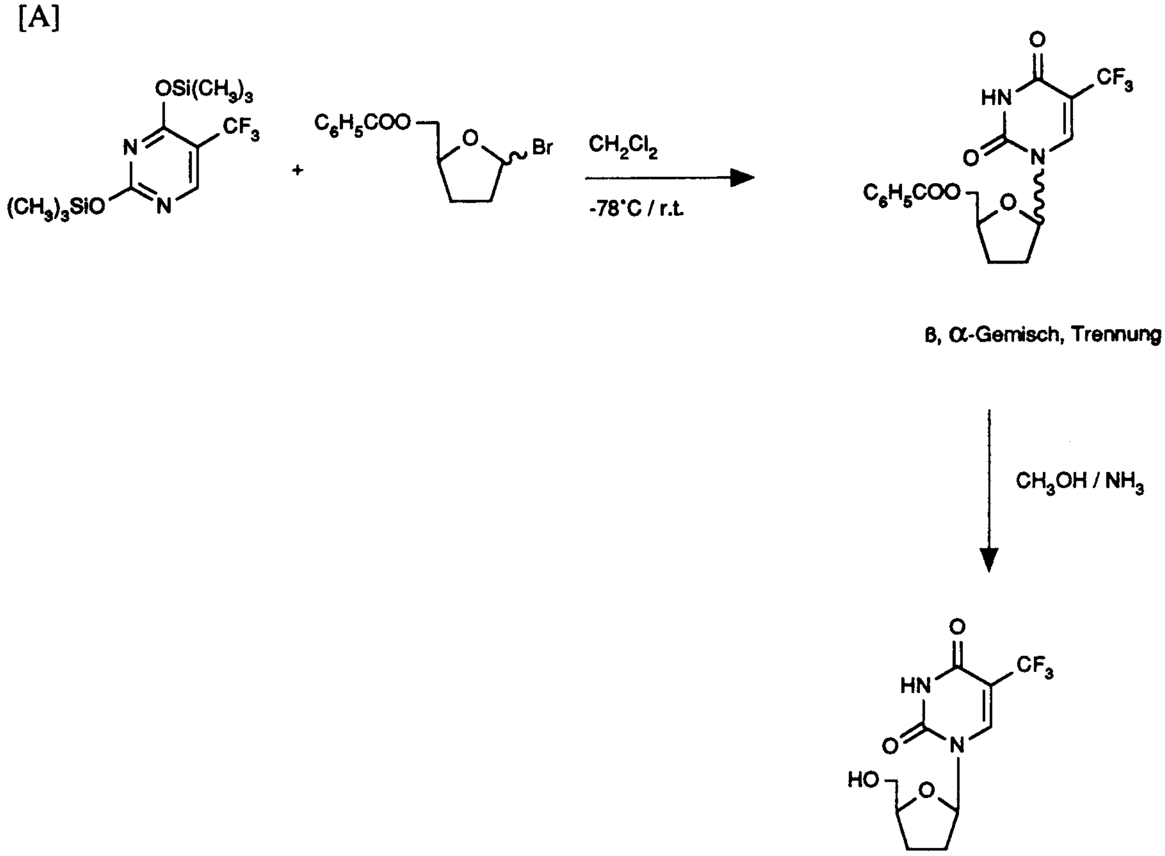
CH₂Cl₂
-78°C / r.t.
β, α-Gemisch, Trennung
CH₃OH / NH₃

[A]

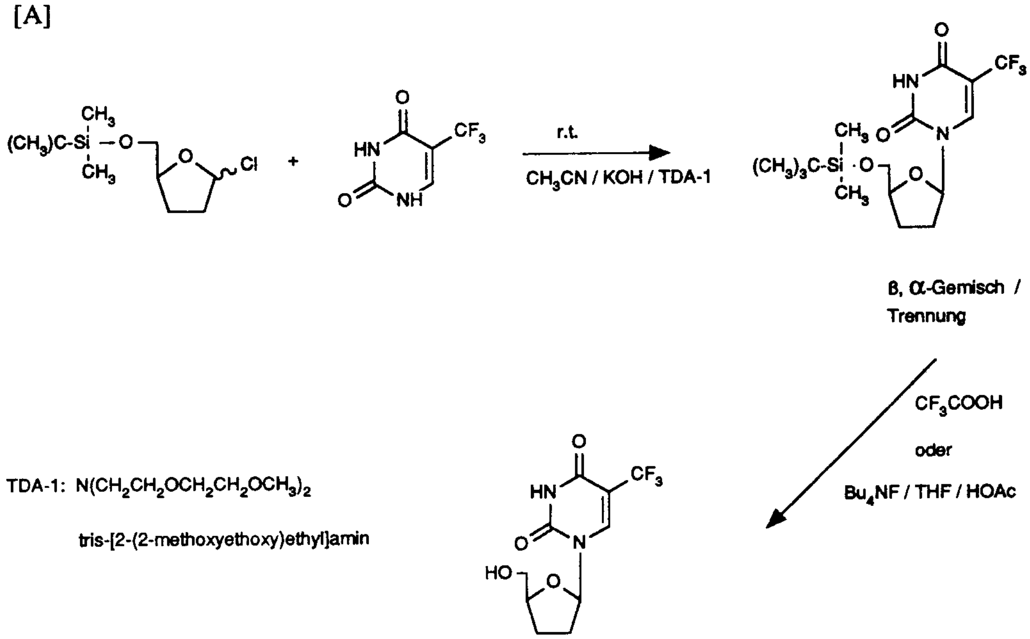
r.t.
CH₃CN / KOH / TDA-1
β, α-Gemisch / Trennung
CF₃COOH
oder
Bu₄NF / THF / HOAc
TDA-1: N(CH₂CH₂OCH₂CH₂OCH₃)₂
tris-[2-(2-methoxyethoxy)ethyl]amin

[B]

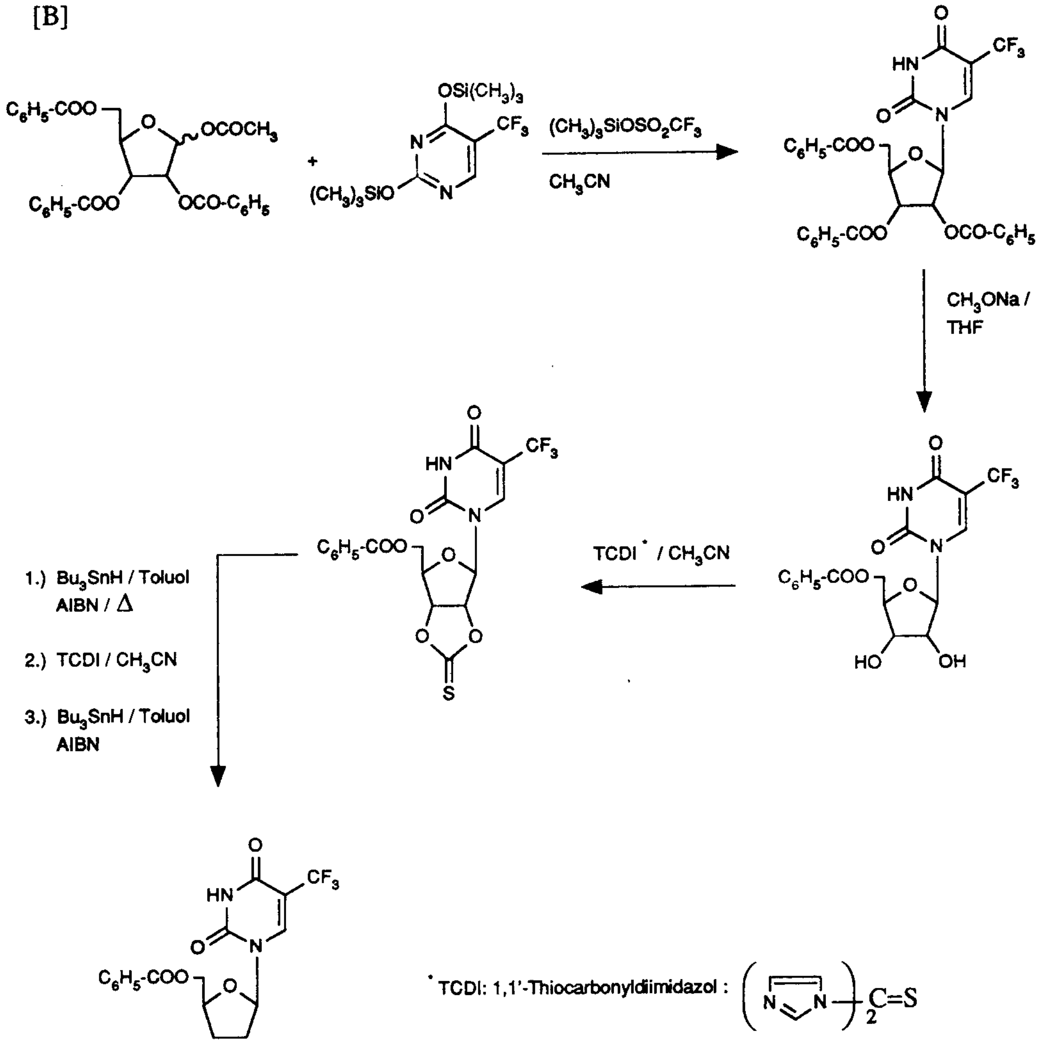
$(CH_3)_3SiOSO_2CF_3$
$CH_3CN$
$CH_3ONa$ / THF
TCDI* / $CH_3CN$
1.) $Bu_3SnH$ / Toluol
AIBN / Δ
2.) TCDI / $CH_3CN$
3.) $Bu_3SnH$ / Toluol
AIBN
* TCDI: 1,1'-Thiocarbonyldiimidazol :

[B]

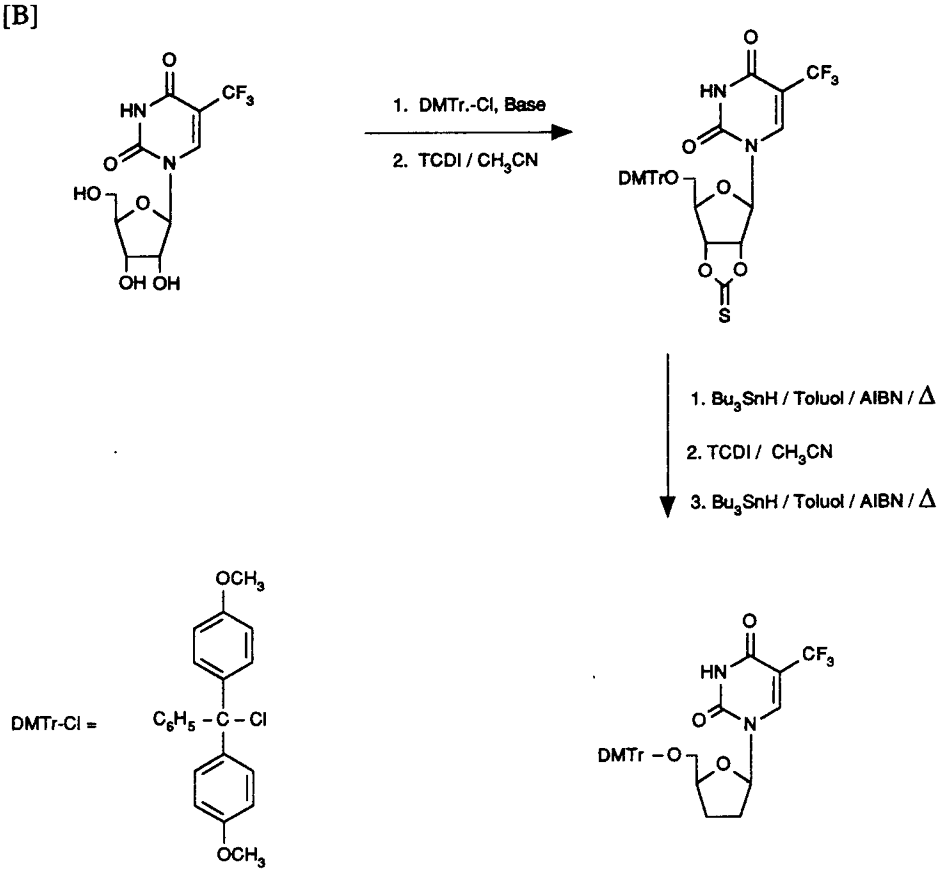
1. DMTr.-Cl, Base
2. TCDI / $CH_3CN$
1. $Bu_3SnH$ / Toluol / AIBN / Δ
2. TCDI / $CH_3CN$
3. $Bu_3SnH$ / Toluol / AIBN / Δ

DMTr-Cl =

[B]

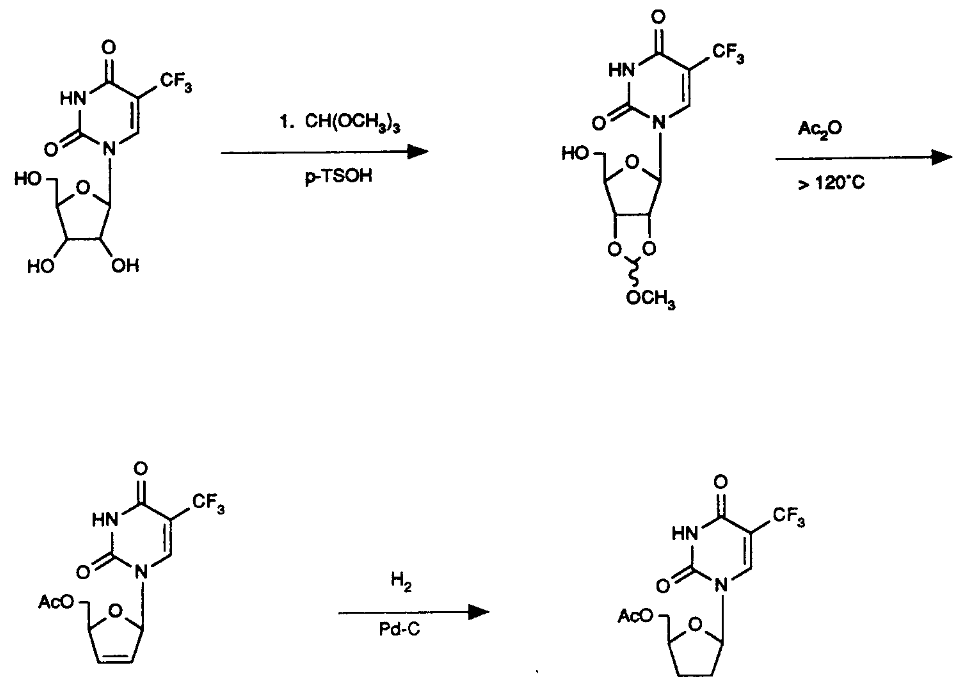
1. $CH(OCH_3)_3$
p-TSOH
$Ac_2O$
> 120°C
$H_2$
Pd-C

[C]

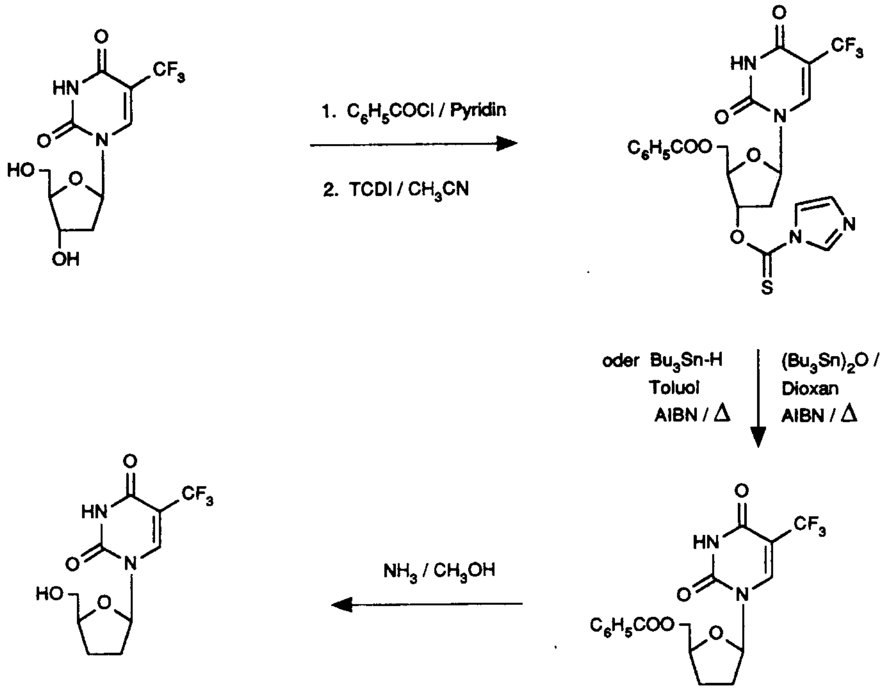
1. $C_6H_5COCl$ / Pyridin
2. TCDI / $CH_3CN$
oder $Bu_3Sn$-H
Toluol
AIBN / Δ
$(Bu_3Sn)_2O$ /
Dioxan
AIBN / Δ
$NH_3$ / $CH_3OH$

[D]

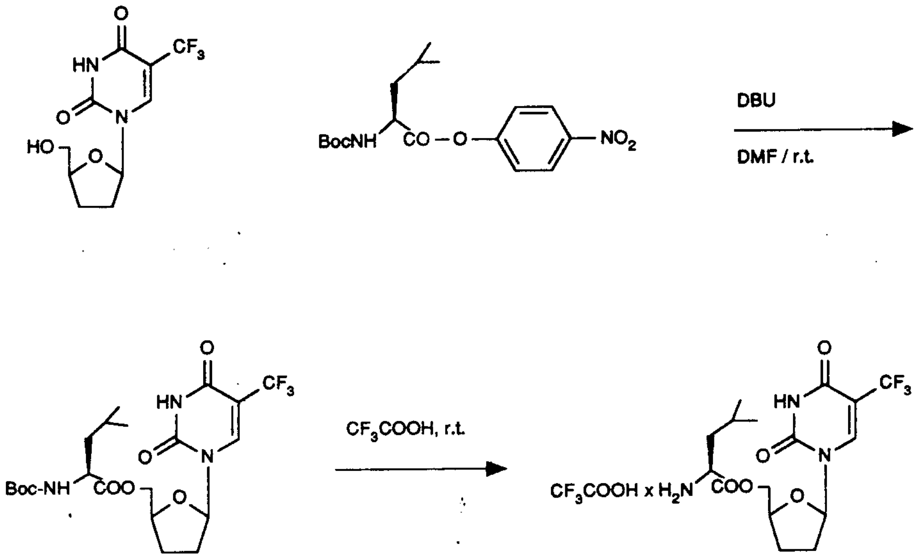


[D]

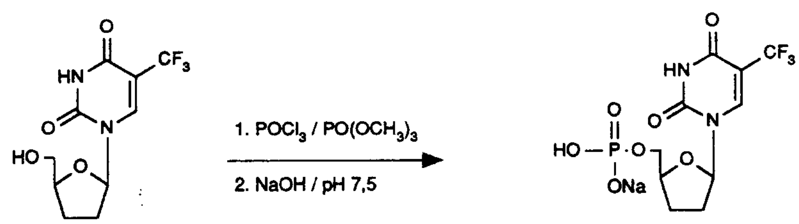

[D]

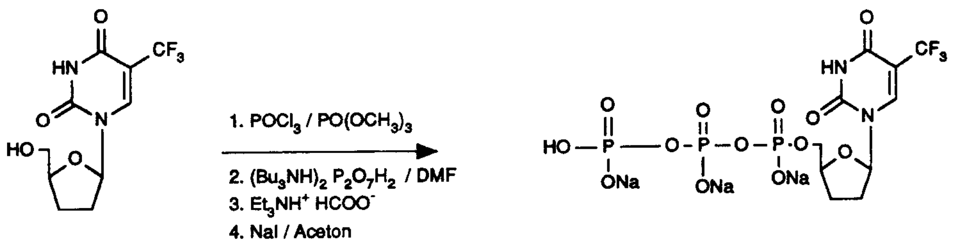


[D]

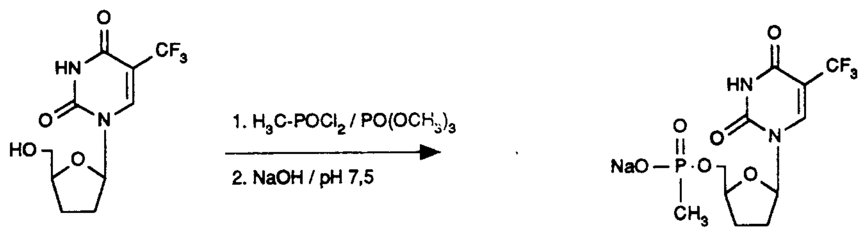


[D]

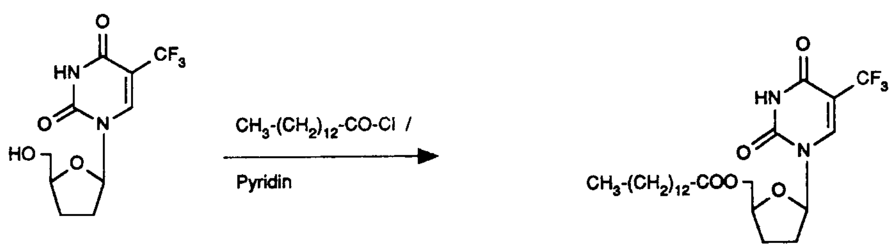


[D]

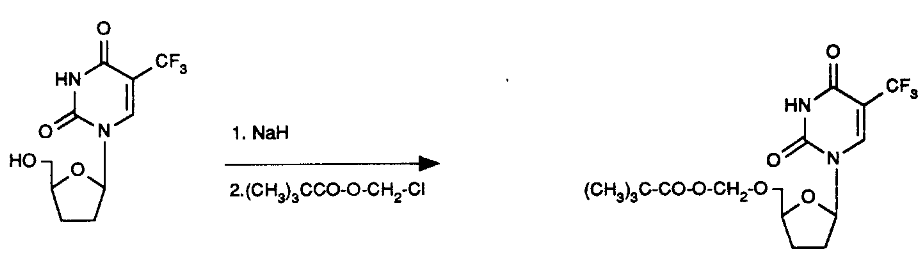


Die Verfahren zur Herstellung der erfindungsgemäßen Verbindungen der allgemeinen Formel (I)

erfolgen teilweise in Analogie zu denen im folgenden aufgeführten literaturbekannten Methoden [vgl. M. Okabe et al., J. Org. Chem. 53, 4780 (1980); V. Farina et al., THL 29, 1239, (1988); F. Seela et al., Heterocycles 29, 2193 (1989); R.R. Webb et al., Nucleosides Nucleotides 7, 147 (1988); EP 357 495; EP 199 451; EP 362 967; L. Vrang et al., Antiviral Res. 7, 139 (1987); EP 366 385; EP 357 495; J.S. Driscoll, J. Med. Chem. 30, 862 (1987); H. Shiragami, J. Org. Chem. 53, 5170 (1988)].

Als Lösemittel für die erfindungsgemäßen Verfahrensvarianten [A] - [D] kommen alle inerten organischen Lösemittel in Frage, die sich unter den Reaktionsbedingungen nicht verändern. Hierzu gehören vorzugsweise Ether wie Diethylether, Dioxan, Tetrahydrofuran, Glykolmonomethylether oder Glykoldimethylether, oder halogenierte Kohlenwasserstoffe wie Dichlormethan, 1,2-Dichlorethan, Trichlormethan oder Tetrachlormethan, oder Amide wie Dimethylformamid, Dimethylacetamid oder Hexamethylphosphorsäuretriamid, oder Kohlenwasserstoffe wie Benzol, Toluol oder Xylol, oder Acetonitril, Nitromethan, Sulfolan, Dimethylsulfoxid, Essigester, oder Alkohole wie Methanol, Ethanol, Isopropanol, Propanol, Butanol, Ethylenglykol oder Pyridin. Ebenso können Gemische der genannten Lösemittel verwendet werden. Bevorzugt sind für die Verfahrensvariante [A] Methylenchlorid und Acetonitril, für die Verfahrensvarianten [B] und [C] Pyridin und Dimethylformamid und für die Verfahrensvariante [D] Dimethylformamid und Dimethylphosphat.

Als Hilfsstoffe für die Umsetzung mit den Verbindungen der allgemeinen Formel (IV) oder (IVa) eignen sich beispielsweise Trimethylsilyltrifluormethansulfonat oder Trimethylsilyltrifluoracetat oder Lewis-Säuren wie beispielsweise Zinn(IV)chlorid, Titan(IV)chlorid, Aluminium(III)chlorid, Quecksilber(II)chlorid oder Zink(II)-chlorid. Bevorzugt sind Trimethylsilyltrifluormethansulfonat, Zinn(IV)chlorid, Titan(IV)chlorid, Aluminium(III)-chlorid oder Quecksilber(II)chlorid.

Die Hilfstoffe werden im allgemeinen in einer Menge von 0,1 mol bis 2,0 mol, bevorzugt in molaren Mengen bezogen auf 1 mol der Verbindungen der allgemeinen Formeln (IV) und (IVa) eingesetzt.

Als Basen eignen sich die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Triethylamin, Pyridin, Picolin, N-Methylmorpholin, N-Methylpiperidin, oder Tris-[2(2-methoxyethoxy)ethyl]-amin, oder 1,8-Diazabicyclo[5.4.0]undec-7-en (DBU) oder 1,5-Diazabicyclo[4.3.0]non-5-en(DBN), oder Ammoniumsulfat.

Die Basen werden im allgemeinen in einer Menge von 1,0 mol bis 3 mol, bevorzugt von 1,0 mol bis 1,2 mol, jeweils bezogen auf 1 mol der Verbindungen der allgemeinen Formeln (IV), (IVa), (VI) oder (IX) eingesetzt.

Die Reaktionstemperaturen können in Bezug auf die jeweilige Verfahrensstufe in einem breiten Bereich variiert werden. Im allgemeinen arbeitet man in einem Bereich von -40 °C bis +170 °C, bevorzugt von -40 °C bis +150 °C.

Alle Umsetzungen in den Verfahren [A] bis [D] können bei normalem, erhöhtem oder bei erniedrigtem Druck durchgeführt werden. Bevorzugt arbeitet man bei einem Druck von 0,5 bis 5 bar.

Die Abspaltung der Silylhydroxyschutzgruppen ($R^6$, gegebenenfalls $R^7$) in den Verbindungen der allgemeinen Formeln (III), (Ia) und (VII) erfolgt nach üblichen Methoden, beispielsweise unter Einwirkung von Fluorsalzen wie beispielsweise Tetrabutylammoniumfluorid, gegebenenfalls in Anwesenheit von Essigsäure und/oder in einem der oben aufgeführten Lösemitteln, bevorzugt in Tetrahydrofuran, oder in Alkoholen, bevorzugt Methanol in Anwesenheit von p-Toluolsulfonsäure.

Die Fluorsalze werden in einer Menge von 1 bis 3 mol und die Essigsäure in einer Menge von 1 bis 5 mol, jeweils bezogen auf 1 mol der Verbindungen der allgemeinen Formeln (III) und (Ia) eingesetzt.

Im Fall, daß $R^7$ zusammen mit dem Sauerstoffatom einen Esterrest bildet, erfolgt die Abspaltung im allgemeinen in einem der oben aufgeführten Alkoholen, wie Methanol oder Ethanol, bevorzugt in Methanol in Anwesenheit von Ammoniak.

Die Abspaltungen erfolgen bevorzugt in einem Temperaturbereich von -10 °C bis +30 °C und unter Normaldruck.

Zur Reduktion der Verbindungen der allgemeinen Formeln (VIII) und (X) eignen sich Zinnhydride, wie beispielsweise Tributylzinnhydrid, Triphenylzinnhydrid oder Zinnoxide wie beispielsweise Tetrabutylzinnoxid. Bevorzugt sind Tributylzinnhydrid und Tetrabutylzinnoxid.

Die Reduktion erfolgt in einem der oben angegebenen Lösemitteln, bevorzugt in Dioxan oder Toluol.

Als Katalysator für die Reduktion eignen sich beispielsweise Azobisisobutyronitril (AIBN) und Dibenzoylperoxid. Bevorzugt ist Azobisisobutyronitril (AIBN).

Der Katalysator wird im allgemeinen in einer Menge von 0,005 mol bis 0,5 mol, bevorzugt von 0,01 mol bis 0,05 mol, bezogen auf jeweils 1 mol der Verbindungen der allgemeinen Formeln (VIII) und (X) eingesetzt.

Die Hydrierung erfolgt in einem der oben aufgeführten Alkohole, beispielsweise Methanol, Ethanol oder

Propanol in Gegenwart eines Edelmetallkatalysators wie Platin, Palladium, Palladium auf Tierkohle oder Raney-Nickel in einem Temperaturbereich von 0°C bis +50°C, bevorzugt von Raumtemperatur bis +40°C bei Normal- oder Überdruck.

Als Lösemittel bei der Darstellung der entsprechenden Mono-, Di- oder Triphosphate und Alkylphosphonate [D] der erfindungsgemäßen Verbindungen der allgemeinen Formel (I) eignen sich neben den oben aufgeführten Lösemitteln auch Phosphorsäureester wie beispielsweise Phosphorsäuretrimethyl- oder Phosphorsäuretriethylester.

Als Basen eignen sich zur Einstellung eines pH-Wertes von 7,5 vorzugsweise Alkalihydroxide wie Natrium- oder Kaliumhydroxid.

Bei der Darstellung der Phosphate wird vorzugsweise Triethylammoniumhydrogencarbonat als Puffer eingesetzt.

Im Fall der Herstellung der Triphosphate der allgemeinen Formel (I) wird bei der Umsetzung mit Phosphoroxychlorid entstehende Dichlorphosphat-Zwischenprodukt nicht zum Monophosphat hydrolysiert, sondern mit Bis(tributylammonium)pyrophosphat direkt zum entsprechenden Triphosphat umgesetzt.

Die Umsetzungen der Verbindungen der allgemeinen Formel (VIII) (X = =S) mit Trialkylphosphiten erfolgt in Analogie zu literaturbekannten Methoden (vgl. J. Org. Chem. 1989, 54, 4780).

Das Abspalten bzw. das Austauschen von Hydroxyschutzgruppen sowie Aminoschutzgruppen erfolgt im allgemeinen nach üblichen Methoden wie sie beispielsweise in Greene, Protective Groups in Organic Synthesis, John Wiley & Sons, 1981, 10 ff und 218 ff beschrieben werden.

Die Herstellung der erfindungsgemäßen Verbindungen der allgemeinen Formel (I), in welcher $R^1$ für einen Aminosäurerest [D] steht, erfolgt durch Abspaltung der jeweiligen Schutzgruppe, gegebenenfalls unter Aktivierung der entsprechenden Säure und Veresterung mit der freien Hydroxymethylfunktion nach den in der Peptidchemie üblichen Methoden [vgl. Houben-Weyl, Eugen Müller, "Methoden der organischen Chemie", Band XV/1 und XV/2, Georg Thieme Verlag, Stuttgart, 1974].

Als Base wird in diesem Fall bevorzugt 1,8-Diazabicyclo[5.4.0]undec-7-en (DBU) eingesetzt.

5-Trifluormethyluracil der Formel (II) ist bekannt.

Die Verbindungen der allgemeinen Formel (III) sind an sich bekannt oder können nach literaturbekannter Methode hergestellt werden [vgl. z.B. Khim Geterotsikl. Soedin, (8), 1128-31, (1), 101-10 und THL, 22 (11), 1029-32].

Die Verbindungen der allgemeinen Formeln (IV) und (IVa) sind an sich bekannt oder können nach literaturbekannten Verfahren hergestellt werden [vgl. z.B. J. Org. Chem. 53, 4780 (1988); THL 29, 1239 (1988) und Heterocycles 29, 2193 (1989); Khim. Geterotsikl. Soedin (1), 101-10 und (8), 1128-31].

Die Verbindungen der allgemeinen Formeln (Ia) und (Ib) sind neu und können nach dem oben aufgeführten Verfahren hergestellt werden.

Die Verbindungen der allgemeinen Formeln (V), (VII) und (IX) sind an sich bekannt oder können nach üblicher Methode hergestellt werden [vgl. J. Med. Chem. 30 (2), 440-4; JP 63 188 696].

Die Verbindungen der allgemeinen Formel (X) sind im Fall, daß L für den Rest $-(C_5)-C_6H_5$ steht teilweise vorbeschrieben (vgl. JP 028 408) oder neu und können dann nach dem oben aufgeführten Verfahren hergestellt werden.

Die Verbindungen der allgemeinen Formel (VI) sind bekannt [vgl. Anal. Biochem. 60, 608 (1974); MSD Book 2, 371 A].

Die Verbindungen der allgemeinen Formeln (XI) und (XII) sind bekannt [vgl. Houben-Weyl, Eugen-Müller, Methoden der organischen Chemie, Band XV/1 und XV/2, Georg Thieme Verlag, Stuttgart 1974 und Amino Acids, G.C. Barrett, London, New York, Chapman and Hall 1985].

Es wurde überraschend gefunden, daß die erfindungsgemäßen Verbindungen der allgemeinen Formel (I) eine außerordentlich starke spezifische Wirkung gegen das Heptatitis B Virus (HBV) besitzen. Der Nachweis wurde in mit Hepatitis B Virus DNA transfizierten Hepatomzellen (HEP G2.2.15) geführt.

Die Ergebnisse der unten aufgeführten Beispiele wurden zu dem in der folgenden Literaturangabe [Sells, M.A.; Chen, M.L., Acs, G., Proc. Natl. Acad. Sci. USA S. 1005 - 1009, Vol. 84 (1987)] beschriebenen HBV Testsystem ermittelt:

Transfizierte Hepatomzellen (HEP G2.2.15) wurden mit verschiedenen Konzentrationen an der jeweiligen Verbindung inkubiert. Durch Behandlung der transfizierten Hepatomzellinie HEP G2.2.15 mit den erfindungsgemäßen Verbindungen konnte das Auftreten der virusspezifischen HBV DNA im Überstand sowie eine Reduktion des $HB_sAg$ Spiegels gezeigt werden. Es wurde weiter gefunden, daß die erfindungsgemäßen Verbindungen zu einer Reduktion der replikativen Intermediate der Hepatitisviren führen. Im Überstand der Zellkulturen wurde nach PEG Fällung der Gehalt an HBV DNA bestimmt. Dies geschah unter Verwendung einer nicht radioaktiv markierten HBV genomischen DNA Probe [Pauly, P., 1982, P.H.D. Thesis, University of Göttingen, F.R.G.; Köchel et al., 1990, EMBL, Accession-Nr. X 51790]. Gleichzeitig

erfolgte der Nachweis der Beeinflussung der $HB_sAg$ Bildung mittels eines kommerziell erhältlichen Elisa Tests. Die angegebenen $IC_{50}$-Werte beziehen sich auf die Substratkonzentrationen, die unter den oben aufgeführten Testbedingungen eine 50%ige Hemmung der HBV DNA Konzentration im Überstand bewirken.

| Bsp.-Nr. | $IC_{50}$(nM) |
|---|---|
| 2 | 0,01 |
| 5 | > 0,25 |

Gleichzeitig erfolgte der möglicherweise vorhandene cytotoxische Einfluß der erfindungsgemäßen Verbindungen mittels Kristallviolettfärbung bzw. über den Einbau von radioaktiv markiertem Thymidin in die zelluläre DNA.

Im Vergleich dazu zeigen die literaturbekannten Substanzen DDA und DDC (vgl. Ueda et al., Virology 169: 213-216 (1989) in dem oben aufgeführten Testsystem folgende $IC_{50}$-Werte: DDA > 100 µm und DDC = 5 µm.

Die Wirkung des Beispiels 2 auf andere Zellen wurde durch Inkubation von HEL-, MEF-Zellen getestet. Es zeigte sich keine Beeinflussung der Vitalität dieser Zellen bis zu einer Konzentration von 250 µM.

Die erfindungsgemäßen Verbindungen stellen wertvolle Wirkstoffe zur Behandlung von virusinduzierten Hepatitiden, hervorgerufen durch Hepdna-Viren, wie Hepatitis-B Viren, Hepatitis C-Viren oder Hepatitis Delta Viren, insbesondere Hepatitis B-Viren, in der Humanmedizin dar.

Als Indikationsgebiete in der Humanmedizin können beispielsweise genannt werden:
Die Behandlung von chronischen Hepatitis B-,-C und Delta- Virusinfektionen und die Behandlung akuter Hepatitis B-, C und Delta-Virusinfektionen, insbesondere von chronischen und akuten Hepatitis B-Virusinfektionen.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Ausgangsverbindungen

Beispiel I

1-(2,3,5-tri-O-Benzoyl-β-D-ribofuranosyl)-5-(trifluormethyl)-uracil

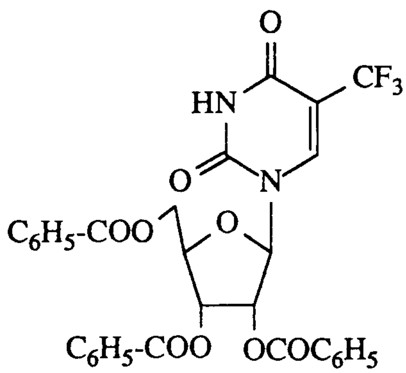

Eine gerührte Suspension von 5,0 g (27,8 mmol) 5-(Trifluormethyl)uracil in 139 ml (0,66 mol) Hexamethyldisilazan wurde mit 211 mg (1,39 mmol) wasserfreiem Ammoniumsulfat versetzt und unter einer Atmosphäre von Stickstoff 5h lang zum Rückfluß erhitzt. Danach wurde das Hexamethyldisilazan in leichtem Vakuum abdestilliert und der Rückstand wurde in 370 ml wasserfreiem Acetonitril gelöst. Man gab 13,31 g (26,40 mmol) 1-O-Acetyl-2,3,5-tri-O-benzoyl-β-D-ribofuranose zu, kühlte auf 0 °C und tropfte innerhalb von 30 min 7,0 ml (36,14 mmol) Trimethylsilyltrifluormethansulfonat zu. Das Kühlbad wurde entfernt, und die Reaktionslösung wurde noch 1h bei Raumtemperatur nachgerührt. Danach wurde in ein kaltes Gemisch aus 170 ml gesättigter $NaHCO_3$-Lösung und 200 ml Ethylacetat eingerührt. Die Phasen wurden getrennt und die Wasserphase wurde mit 2 x 200 ml Ethylacetat extrahiert. Die vereinigten Extrakte wurden über $MgSO_4$ getrocknet, das Lösemittel wurde im Vakuum abgedampft und der Rückstand wurde an 200 g Kieselgel (Toluol : Ethylacetat 4:1) chromatographiert. Man erhielt 15,3 g (88%) der Titelverbindung als farblosen Schaum.

$R_f$ = 0,35 (Toluol: Ethylacetat 4:1)

MS (FAB) m/z = 625 $(M+H)^+$

<u>Beispiel II</u>

1-(5-O-Benzoyl-β-D-ribofuranoyl)-5-(trifluormethyl)-uracil

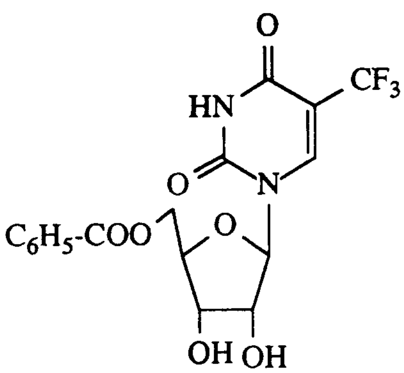

In Analogie zu S.N. Shino et al., Tetrahedron <u>41</u>, 5503 (1985) gab man zu einer gerührten Suspension von 2,36 g (43,6 mmol) Natriummethanolat in 200 ml wasserfreiem THF bei 0 °C portionsweise 10,90 g (17,50 mmol) der getrockneten Verbindung aus Beispiel I. Das Kühlbad wurde entfernt, man rührte 1h bei 0 °C nach und neutralisierte (pH 6,5) die Reaktionsmischung durch Zugabe von Ionenaustauscher Dowex 50 W-X 4 ($H^+$-Form). Der Ionenaustauscher wurde durch Filtration abgetrennt und mit 3 x 20 ml Ethanol gewaschen. Das Filtrat wurde im Vakuum zur Trockene eingeengt und an 285 g Kieselgel (Dichlormethan : Methanol 95:5) chromatographiert. Man erhielt 4,48 g (62%) der Titelverbindung als farblose Kristalle.

Schmp.: 186 °C

$R_f$ = 0,40 (Dichlormethan: Methanol 9:1)

MS (FAB) m/z = 417 $(M+H)^+$

Beispiel III

1-(5'-O-Benzoyl-β-D-ribofuranosyl)-5-trifluormethyl-uracil-2',3'-thionocarbonat

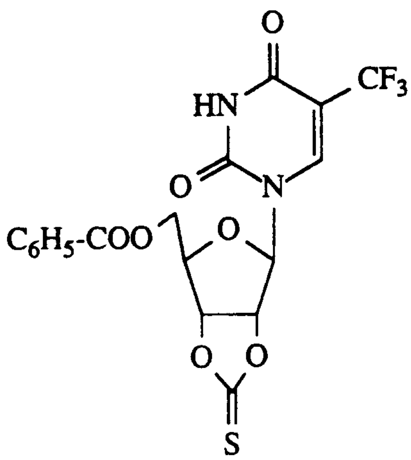

Eine Lösung von 3,33 g (8,0 mmol) der Verbindung aus Beispiel II in 150 ml wasserfreiem Acetonitril wurde portionsweise mit 3,60 g (18,16 mmol) 1,1'-Thiocarbonyldiimidazol versetzt und 2 h bei Raumtemperatur gerührt. Danach wurde das Lösemittel im Vakuum abgedampft und der Rückstand durch Filtration an 100 g Kieselgel (Toluol : Ethylacetat 3:2) gereinigt. Man erhielt 3,26 g (89% der Theorie) der Titelverbindung als farbloses Pulver.
Schmp.: 133 °C (Zers.)
$R_f$ = 0,71 (Ethylacetat)
IR (KBr): $\gamma$ = 3188, 3073, 1697, 1469, 1379, 1326, 1270, 1144, 1097, 1012, 720 $cm^{-1}$ .
MS (FAB) m/z = 459 $(M+H)^+$, 475 $(M+Na)^+$

Beispiel IV

5'-O-Benzoyl-2',3'-didehydro-2',3'-didesoxy-5-(trifluormethyl)-uridin

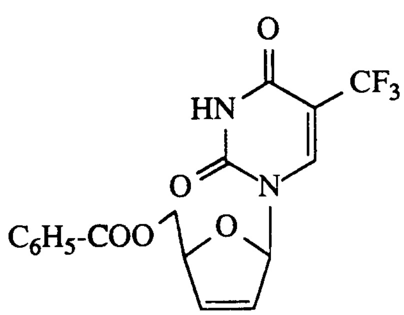

Zu 75 ml (66 equiv.) frisch destilliertem unter einer Stickstoffatmosphäre auf 130 °C vorgeheiztem Triethylphosphit gab man auf einmal 3,00 g (6,55 mmol) 1-(5'-O-Benzoyl-β-D-ribofuranosyl)-5-trifluormethyl-uracil-2',3'-thionocarbonat (Beispiel III) und erwärmte 1,5 h auf 130 °C (**exakt** !). Danach wurde die Reaktionsmischung abgekühlt, mit 10 ml Toluol versetzt und am Rotationsverdampfer zur Trockene eingeengt. Dieser Vorgang wurde noch zweimal wiederholt, um letzte Reste von Triethylphosphit zu entfernen. Der noch toluolfeuchte zurückbleibende Kristallbrei wurde mit 20 ml Ether versetzt, und gut durchgerührt, abgesaugt, mit wenig Ether gewaschen und im Hochvakuum über Sicapent getrocknet. Durch

Aufkonzentrieren des Filtrats erhielt man eine weitere Menge der Titelverbindung. Gesamtausbeute 1,59 g (63%) farblose Kristalle. Durch Chromatographie der Mutterlauge an 40 g Kieselgel (Toluol : Ethylacetat 7:3) erhielt man weitere 230 mg (9%) der Titelverbindung.
Schmp.: 157 °C
$R_f$ = 0,24 (Toluol : Ethylacetat 7:3)
MS (FAB-): m/z = 381 $(M-H)^-$.
IR (KBr): $\nu$ = 3434, 3181, 3065, 1703, 1462, 1327, 1280, 1147, 1120, 1098, 1043, 708 $cm^{-1}$

Beispiel V

2'-Desoxy-5'-O-(4-methylbenzoyl)-5-(trifluormethyl)-uridin

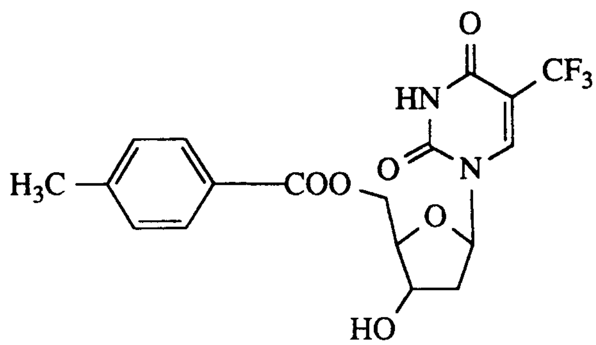

Zu einer auf 0 °C gekühlten Lösung von 593 mg (2,00 mmol) 2'-Desoxy-5-(trifluormethyl)-uridin (Trifluridin®) in 12 ml wasserfreiem Pyridin unter einer Stickstoffatmosphäre tropfte man innerhalb von 2 h eine Lösung von 290 µl (2,20 mmol) p-Toluoylchlorid in 12 ml Pyridin. Danach rührte man 15 min bei 0 °C und tropfte dann zur Vernichtung überschüssigen p-Toluoylchlorids 10 ml wasserfreies Methanol zu. Das Kühlbad wurde entfernt und die Reaktionsmischung im Vakuum eingeengt und der Rückstand an 85 g Kieselgel (Dichlormethan : Methanol 9:1) chromatographiert. Man erhielt 71% der Titelverbindung als farblose Kristalle.
Schmp.: 214 °C
$R_f$ = 0,31 (Dichlormethan: Methanol 9:1)
MS (FAB): m/z = 415 $(M+H)^+$

Beispiel VI

2'-Desoxy-3'-O-(Imidazolyl-thiocarbonyl)-5'-O-(4-methylbenzoyl)-5-(trifluormethyl)-uridin

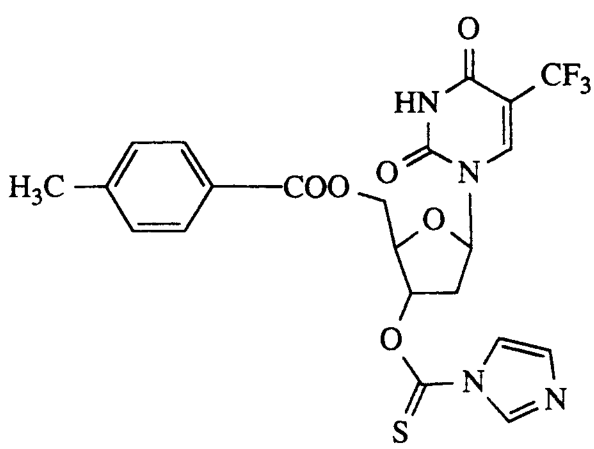

Eine Suspension von 410 mg (0,99 mmol) der Verbindung aus Beispiel V in 15 ml wasserfreiem Acetonitril wurde mit 353 mg (1,98 mmol) 1,1'-Thionylcarbonyldiimidazol versetzt und 25 h bei Raumtemperatur gerührt. Das Lösemittel wurde ohne zu heizen im Vakuum abgedampft und der Rückstand wurde in ein Gemisch von 10 ml Ethylacetat, 10 ml gesättigter NaCl-Lösung, 5 ml gesättigter $NaHCO_3$-Lösung und 5 g Eis eingerührt. Die organische Phase wurde abgetrennt und die wäßrige Phase mit 10 ml Ethylacetat extrahiert. Die vereinigten Extrakte wurden über $MgSO_4$ getrocknet, das Lösemittel im Vakuum abgedampft und der Rückstand wurde mit wenig Ether verrieben. Man erhielt 474 mg (91%) des Thiocarbonylimidazolids als farbloses Pulver.
Schmp.: 87 °C
$R_f$ = 0,30 (Dichlormethan : THF 4:1)
MS (FAB) m/z = 525 $(M+H)^+$
IR (KBr): $\nu$ = 3424, 1700, 1128 $cm^{-1}$

Herstellungsbeispiele

Beispiel 1

$\alpha,\beta$-5'-(tert.Butyldimethylsilyl)-2',3'-didesoxy-5-(trifluormethyl)-uridin (Anomerengemisch)

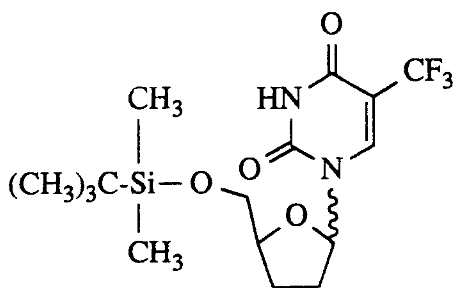

Ein gerührtes Gemisch von 0,54 g (3,0 mmol) 5-(Trifluormethyl)-uracil (getrocknet 24 h bei 80 °C) und 21,1 ml (100 mmol) Hexamethyldisilazan wurde in Gegenwart von 20 mg (0,15 mmol) Ammoniumsulfat unter einer Atmosphäre von Stickstoff 1 h auf 140 °C erhitzt. Die Klare Lösung durfte abkühlen und die flüchtigen Bestandteile wurden im Vakuum abdestilliert. Das zurückbleibende Öl wurde mit 5 ml wasserfreiem Toluol versetzt und die flüchtigen Bestandteile wurden erneut im Vakuum abdestilliert. Das zurückbleibende Öl wurde in 40 ml wasserfreiem Acetonitril gelöst, mit 0,82 g (3,0 mmol) 1-O-Acetyl-5-(tert.butyldimethylsilyl)-2',3'-didesoxyribose [M. Okabe et al., J. Org. Chem. 53, 4780 (1988)] versetzt und auf -10 °C gekühlt. Dazu gab man tropfenweise 0,64 ml (3,3 mmol) frisch destilliertes Trimethylsilyltrifluormethansulfonat und rührte 30 min bei -10 °C nach. Danach wurde die Reaktionslösung in ein Gemisch aus 50 ml gesättigter $NaHCO_3$-Lösung und 50 ml Ethylacetat eingerührt. Die organische Phase wurde abgetrennt und die wässrige Phase mit 25 ml Ethylacetat extrahiert (2 mal). Die vereinigten organischen Phasen wurden über $MgSO_4$ getrocknet. Abdampfen des Lösemittels im Vakuum ergab 1,15 g (97%) der Titelverbindung als Öl (Anomerengemisch), welches ohne weitere Reinigung weiter umgesetzt wurde.
$R_F$ = 0,83, 0,76 (Ethylacetat)
MS (FAB) m/z = 395 $(M+H)^+$.

Beispiel 2 und Beispiel 3

$\beta$-2',3'-Didesoxy-5-(trifluormethyl)-uridin (Beispiel 2)

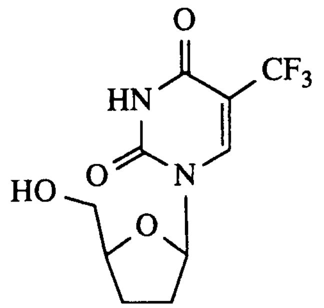

und

α-2',3'-Didesoxy-5-(trifluormethyl)-uridin (Beispiel 3)

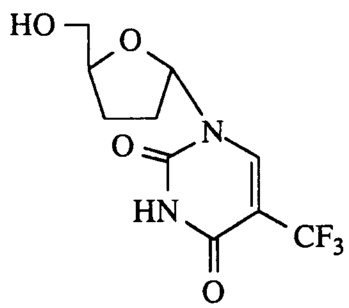

Eine Lösung von 1,15 g (3,0 mmol) des in Beispiel 1 erhaltenen Anomerengemisches und 628 mg (3,3 mmol) p-Toluolsulfonsäuremonohydrat in 8 ml Methanol und 1,2 ml Wasser wurde 30 min bei Raumtemperatur gerührt. Danach gab man 0,84 ml (6,0 mmol) Triethylamin zu. Nach Abdampfen des Lösemittels im Vakuum und Chromatographie des Rückstandes an 130 g Kieselgel (Toluol:Ethylacetat 1:1) erhielt man 303 mg (36%) des unpolaren β-Isomers (Beispiel 2) als farblose Kristalle.
Schmp.: 165 ° C
$R_f$ = 0,46 (Ethylacetat); 0,27 (Dichlormethan:Methanol 9:1)
HPLC-Reinheit >99,5%
MS (DCI, $NH_3$) m/z = 281 $(M+H)^+$, 298 $(M+NH_4)^+$
IR (KBr): 3466, 3192, 3074, 1706, 1461, 1410, 1334, 1286, 1141, 1092, 1066, 1040, 614 $cm^{-1}$.
$^1$H-NMR (250 MHz, $CD_3OD$): δ = 1,90 - 2,50 (m, 4H, H-2', H-3'); 3.70, 3,98 (AB, 2H, J = 12,5 Hz, 2,5 Hz, H-5'); 4,20 (m, 1H, H-4'); 6.01 (dd, 1H, J = 7,0 Hz, 1,5 Hz, H-1'); 8,98 (s, 1H, H-6).
$C_{10}H_{11}F_3N_2O_4$ (280,21)
Ber.: C 42,87 H 3,96 N 10,00
Gef.: C 42,7 H 4,0 N 9,9
Außerdem erhielt man 270 mg (32%) des polaren α-Isomers (Beispiel 3) als farblosen Schaum.
$R_f$ = 0,36 (Ethylacetat)
HPLC-Reinheit = 99%
MS (DCI, $NH_3$) m/z = 281 $(M+H)^+$, 298 $(M+NH_4)^+$
IR (KBr): 3448, 1702, 1473, 1412, 1334, 1276, 1129, 612 $cm^{-1}$
$^1$H-NMR (250 MHz, $CD_3OD$): δ = 1,85 - 2,00 (m, 3H, H-2', H-3'); 2,51 (m, 1H, H-2'); 3,54 (dd, 1H, J = 12,5 Hz, 6 Hz, H-5'); 3,66 (dd, 1H, J = 12,5 Hz, 3,5 Hz H-5'); 4,50 (m, 1H, H-4'); 6,00 (dd, 1H, J = 6,5 Hz, 5,5 Hz, H-1'); 8,00 (s, 1H, H-6).

Außerdem kann die Verbindung aus Beispiel 2 folgendermaßen hergestellt werden:
Eine Lösung von 25 mg (0,45 mmol) Natriummethanolat in 11 ml wasserfreiem Methanol wurde mit 150 mg (0,4 mmol) 5'-O-Benzoyl-2',3'-didesoxy-5-trifluormethyluridin versetzt und 1,6 h bei Raumtemperatur gerührt. Danach wurde die Reaktionsmischung durch Zugabe von Ionentauscher Dowex 50 X4 ($H^+$-Form), neutralisiert, der Ionentauscher wurde durch Filtration abgetrennt und das Filtrat im Vakuum zur Trockne eingedampft. Durch Verreiben mit Ether/Pentan erhielt man 104 mg (93%) der Titelverbindung als farblose

Kristalle.
Schmp.: 165 ° C

Beispiel 4

β-5'-(2,2-Dimethylpropanoyloxymethyl)-2',3'-didesoxy-5-(trifluormethyl)-uridin

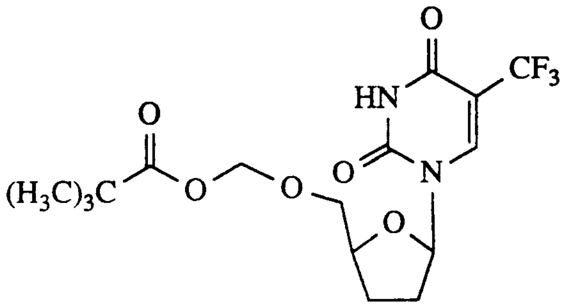

Zu einer auf 0 ° C gekühlten, gerührten Suspension von 18 mg (0,59 mmol) Natriumhydrid (80%ig in Öl) in 1 ml wasserfreiem DMF tropfte man langsam eine Lösung von 150 mg (0,54 mmol) β-2',3'-Didesoxy-5-trifluormethyluridin in 10 ml wasserfreiem DMF. Nach Beendigung der Wasserstoffentwicklung (ca. 2 h) gab man eine Lösung von 89 mg (0,59 mmol) Pivaloyloxymethylchlorid in 1 ml wasserfreiem DMF zu, entfernte das Kühlbad und rührte 70 h bei Raumtemperatur nach. Der größte Teil des Lösemittels wurde im Vakuum abgedampft, wobei darauf geachtet wurde, daß die Temperatur nicht über 35 ° C stieg. Der Rückstand wurde in ein Gemisch aus 10 ml Wasser und 10 ml Ethylacetat aufgenommen. Die organische Phase wurde abgetrennt und die Wasserphase mit 10 ml Ethylacetat (2 mal) extrahiert und die vereinigten organischen Extrakte wurden über $MgSO_4$ getrocknet. Nach Abdampfen des Lösemittels im Vakuum und Chromatographie des Rückstandes an 24 g Kieselgel (Toluol : Ethylacetat 7:3) erhielt man 134 mg (66%) der Titelverbindung als farblosen Schaum.
$R_f$ = 0,58 (Ethylacetat)
MS (DCI, $NH_3$) m/z = 395 $(M+H)^+$, 412 $(M+NH_4)^+$
IR (KBr): $\gamma$ = 3530, 2983, 1737, 1685, 1473, 1288, 1133, 785 $cm^{-1}$.

Beispiel 5

β-5'-(1-Ethoxycarbonyloxy-eth-1-yl)-2',3'-didesoxy-5-(trifluormethyl)-uridin

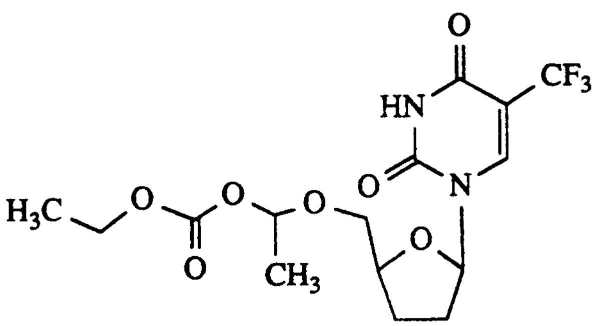

Wie für Beispiel 4 beschrieben erhielt man aus 100 mg (0,36 mmol) β-2',3'-Didesoxy-5-(trifluormethyl)-uridin und 62 (0,40 mmol) 1-Chlor-1-ethoxycarbonyloxyethan nach Chromatographie des Rohproduktes an 8 g Kieselgel (Toluol : Ethylacetat 1:1) 62 mg (47%) der Titelverbindung als farblose Kristalle.
Schmp.: 114 ° C
$R_f$ = 0.33 (Toluol : Ethylacetat 1:1)
MS (DCI, $NH_3$) m/z = 353 $(C_{13}H_{15}F_3N_2O_6+H)^+$, 370 $(M+NH_4)^+$

IR (KBr): $\gamma$ 3019, 1704, 1277, 1220, 1212 $cm^{-1}$.

Beispiel 6

$\beta$-5-Hemisuccinyl-2',3'-didesoxy-5-(trifluormethyl)-uridin

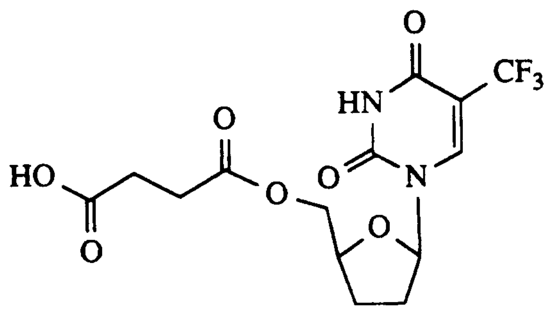

Eine Lösung von 100 mg (0,36 mmol) $\beta$-2',3'-Didesoxy-5-(trifluormethyl)-uridin in 4 ml wasserfreiem Pyrid in wurde mit 54 mg (0,54 mmol) Bernsteinsäureanhydrid versetzt und 65 h bei Raumtemperatur gerührt. Nach Abdampfen des Lösemittels im Vakuum und Chromatographie des Rohprodukts an 30 g Kieselgel (Dichlormethan : Methanol 9:1) erhielt man 96 mg (70%) der Titelverbindung als hellen Feststoff.
$R_f$ = 0,19 (Dichlormethan : Methanol)
MS (FAB) m/z = 381 $(M+H)^+$.

Beispiel 7

5'-O-[(tert.Butoxycarbonyl)-L-leucyl)]-2',3'-didesoxy-5-(trifluormethyl)-uridin

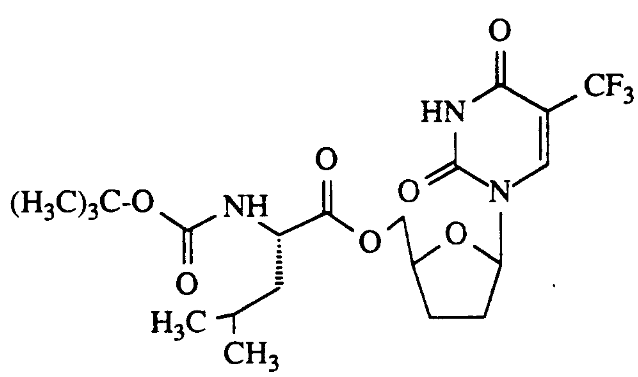

Eine gerührte Lösung von 150 mg (0,54 mmol) $\beta$-2',3'-Didesoxy-5-(trifluormethyl)-uridin und 634 mg (1,08 mmol) (tert.Butoxycarbonyl)-L-leucin-4-nitrophenylester [Houben-Weyl 15/2 S. 33] in 3 ml wasserfreiem DMF wurde mit 162 µl (1,08 mmol) DBU versetzt und 48 h bei Raumtemperatur gerührt. Danach wurde das Lösemittel im Vakuum weitgehend abgedampft und der Rückstand wurde in 40 ml Ethylacetat aufgenommen. Die organische Phase wurde mit je 20 ml 10%iger $Na_2CO_3$-Lösung, Wasser und gesättigter NaCl-Lösung gewaschen und über $MgSO_4$ getrocknet. Nach Abdampfen des Lösemittels im Vakuum und Chromatographie des Rückstands an 40 g Kieselgel (Toluol : Ethylacetat 1:1) erhielt man 195 mg (73%) der Titelverbindung als farblosen Schaum.
$R_f$ = 0,69 (Ethylacetat)
MS (FAB) m/z = 494 $(M+H)^+$
IR (KBr): $\gamma$ = 2965, 1703, 1470, 1369, 1275, 1162, 1091, 1036 $cm^{-1}$.

Beispiel 8

5'-O-(L-Leucyl)-2',3'-didesoxy-5-(trifluormethyl)uridin-Hydrochlorid

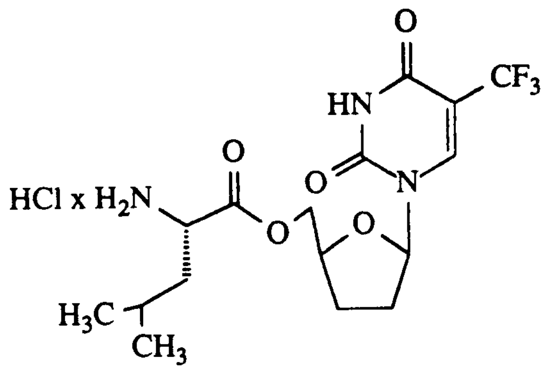

Eine auf +5°C gekühlte, gerührte Suspension von 99 mg (0,2 mmol) der Verbindung aus Beispiel 7 in 1 ml wasserfreiem Dioxan wurde langsam mit 1 ml einer 4 N Lösung gasförmigen Chlorwasserstoffs in wasserfreiem Dioxan versetzt und 30 min bei +5°C und 4 h bei Raumtemperatur gerührt. Danach gab man 5 ml Toluol zu und engte im Vakuum ein. Dieser Vorgang wurde wiederholt, dann wurde der Rückstand mit 5 ml Ether verrieben, abgesaugt und im Hochvakuum über KOH getrocknet. Man erhielt 76 mg (96%) der Titelverbindung als farbloses Pulver.
$R_f$ = 0,44 (Acetonitril : Wasser 9:1)
MS (FAB) m/z = 394 $(M+H)^+$
IR (KBr): $\gamma$ = 3433, 2965, 1701, 1472, 1413, 1276, 1132, 1090, 1038, 616 $cm^{-1}$.

Beispiel 9

2',3'-Didesoxy-5-(trifluormethyl)-uridin-5'-monophosphat (Natriumsalz)

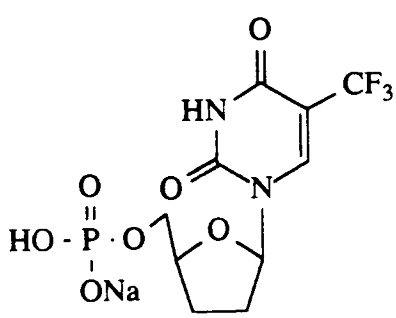

Eine auf -10°C gekühlte Suspension von 100 mg (0,36 mmol) $\beta$-2',3'-Didesoxy-5-(trifluormethyl)-uridin (Beispiel 2) in 1 ml Trimethylphosphat wurde mit 67 $\mu$1 (0,71 mmol - 2 equiv.) Phosphoroxychlorid versetzt und 24 h bei 0°C gerührt. Danach wurde in 10 ml Eiswasser gegossen und mit 2 H NaOH pH 7,5 eingestellt. Die wässrige Lösung wurde durch Extraktion mit Dichlormethan und Ether von organischem Material befreit, im Vakuum auf ein Volumen von ca. 1 ml konzentriert und an 40 ml Diaion HP-20 (Wasser) chromatographiert. Die produkthaltigen Fraktionen wurden gesammelt, gefriertgetrocknet, in 1 ml Wasser aufgenommen und in einer Lobar-Fertigsäule Größe B (310-25) Lichroprep., RP-8 (40-63 $\mu$m) mit Wasser chromatographiert. Die produkthaltigen Fraktionen wurden gesammelt und gefriergetrocknet. Man erhielt 129 mg (93%) der Titelverbindung als farbloses Lyophilisat.
$R_f$ = 0,33 (Acetonitril : Wasser 8:2)
IR (KBr): $\gamma$ = 3430, 1702, 1066, 860, 545 $cm^{-1}$.

Beispiel 10

2',3'-Didesoxy-5-(trifluormethyl)-uridin-5'-triphosphat, Trinatriumsalz

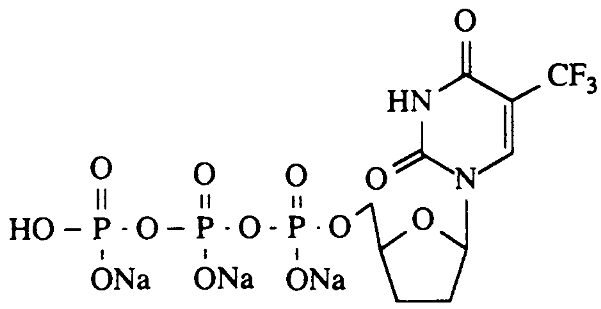

Eine auf 0°C gekühlte Lösung von 150 mg (0,54 mmol) 2',3'-Didesoxy-5-(trifluormethyl)-uridin (Beispiel 1) in 1 ml Trimethylphosphat wurde mit 0,11 ml (1,2 mmol) Phosphoroxychlorid versetzt und 18 h bei 0°C gerührt. Danach wurden bei 0°C 0,57 ml (2,40 mmol) Tributylamin und 6,7 ml (2,70 mmol) einer 0,4 M-Lösung von bis-(Tributylammonium)pyrophosphat [D.E. Hoard et al., J. Am. Chem. Soc., 87, 1785 (1965)] in DMF zugetropft. Nach 25 min bei 0°C wurde die Reaktion durch Zugabe von 9 ml 1 M wässrigem Tributylammoniumhydrogencarbonat (TEAB) Puffer abgebrochen und ein pH-Wert von 7,4 eingestellt. Die Wasserphase wurde mit 5 x 50 ml Dichlormethan extrahiert und dann durch Zugabe von 4 ml Dowex 50 W-X 4 ($H^+$-Form) auf pH 1,5 gestellt. Der Ionenaustauscher wurde durch Filtration abgetrennt und die klare Filtratlösung wurde an 40 ml Sephadex A 25 (Pharmacia), welches mit 0,1 M TEAB äquilibiert war, chromatographiert Die Säule wurde mit einem zunehmenden Gradienten (0,1 M pro 20 ml Eluat) an TEAB-Puffer von 0,1 M bis 0,5 M eluiert. Die produkthaltigen Fraktionen wurden am Rotationsverdampfer zur Trockene eingeengt, mit wenig Methanol versetzt und abermals zur Trockene eingeengt. Dieser Vorgang wurde noch 5 mal wiederholt, dann wurde eine Lösung des Rohproduktes in 3 ml Methanol mit 3 ml (3 mmol) einer 1 M Lösung von Natriumiodid in Aceton versetzt, wodurch das Triphosphat als Natriumsalz ausfiel. Der erhaltene Niederschlag wurde noch 3 mal mit 5 ml Aceton gewaschen (Zentrifuge) und dann im Hochvakuum über $P_4O_{10}$ (Phosphorpentoxid) getrocknet. Man erhielt 169 mg (54% der Theorie) der Titelverbindung als farblosen Feststoff.

$R_f$ = 0,18 (Acetonitril : Wasser 4:1)

IR (KBr): $\gamma$ = 3433, 1706, 1478, 1277, 1103, 997, 896, 769 $cm^{-1}$.

Beispiel 11

2',3'-Didesoxy-5-(trifluormethyl)-uridin-5'-methylphosphonat

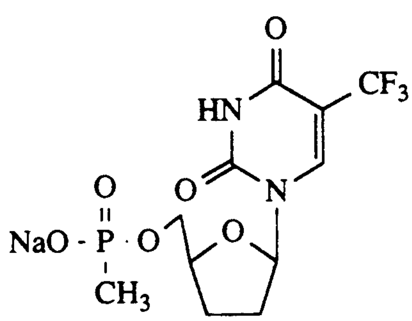

Eine auf 0°C gekühlte Lösung von 100 mg (0,36 mmol) 2',3'-Didesoxy-5-(trifluormethyl)-uridin (Beispiel 1)in1 ml Trimethylphosphat wurde mit 71 mg (0,54 mmol) Methanphosphonsäuredichlorid versetzt und 28 h bei -22°C stehen gelassen. Danach wurde die Reaktionsmischung mit 8 ml wasserfreiem Dichlormethan versetzt, in 25 ml kaltes Wasser gegeben und durch Zugabe von 1 N NaOH-Lösung auf pH 8,0 gestellt. Die Wasserphase wurde mehrmals mit Dichlormethan extrahiert und dann gefriergetrocknet. Nach Chromatographie des Rohprodukts an Diaion HP 20 (Wasser) und Gefriertrocknen der produkthaltigen Fraktionen erhielt man 63 mg (48%) der Titelverbindung als farbloses Lyophilisat.

$R_f$ = 0,32 (Acetonitril : Wasser 4:1)

IR (KBr):$\gamma$ = 3448, 1629, 1467, 1201, 1029, 949, 808 $cm^{-1}$
MS (SIMS) m/z = 369 $(M+Na)^+$.

Beispiel 12

$\beta$-2',3'-Didesoxy-5-(trifluormethyl)-uridin-natriumsalz

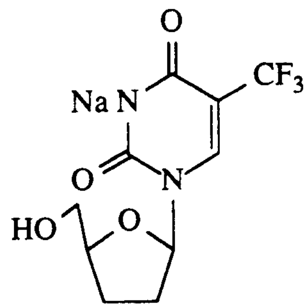

Eine auf 0°C gekühlte, gerührte Suspension von 105 mg (3,50 mmol) Natriumhydrid (80% in Öl) in 5 ml wasserfreiem THF wurde tropfenweise mit einer Lösung von $\beta$-2',3'-Didesoxy-5-trifluormethyl-uridin in 10 ml THF versetzt. Das Kühlbad wurde entfernt und die Mischung wurde bis zum Ende der Gasentwicklung (ca. 10 min.) gerührt. Danach wurde das Lösemittel im Vakuum abgedampft und der Rückstand in 20 ml destilliertem Wasser gelöst. Die Wasserphase wurde mit 10 ml Ethylacetat extrahiert (3 mal), im Vakuum von Lösemittelresten befreit, durch einen Millipore Membranfilter (0,22 $\mu$) filtriert und gefriergetrocknet.
Man erhielt 971 mg (90%) der Titelverbindung als farbloses, gut wasserlösliches Lyophilisat.
$R_f$ = 0,46 (Ethylacetat)
HPLC-Reinheit: >99%

Beispiel 13

5'-O-Benzoyl-2',3'-didesoxy-5-(trifluormethyl)-uridin

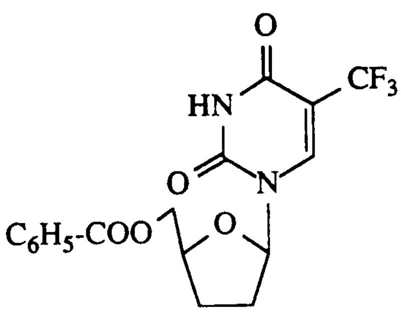

Weitere Darstellungsmöglichkeiten:

Methode A:

Zu einer Suspension von 120 mg vorhydriertem Palladium auf Kohle (10%) in 40 ml THF (p.a.) gab man auf einmal 410 mg (1,07 mmol) der Verbindung aus Beispiel IV und hydrierte 1 h bei 20°C und 1 atm Wasserstoff (Bei Bedarf werden weitere 60 mg des Katalysators zugegeben und man hydriert noch 1 h bei Raumtemperatur). Nach Abschluß der Reaktion (DC) wurde der Katalysator durch Filtration an ca. 10 g mit THF gewaschenem Kieselgur abgetrennt, das Filtrat wurde im Vakuum eingeengt und der Rückstand an 25 g Kieselgel (Toluol : Ethylacetat 3:2) chromatographiert. Man erhielt 304 mg (74%) der Titelverbindung als farblosen Schaum, welcher aus Ether/Pentan langsam kristallisierte.

Schmp.: 93 ° C
$R_f$ = 0,35 (Toluol:Ethylacetat 3:2);
$R_f$ = 0,56 (Ethylacetat)
MS (DCI, $NH_3$): m/z = 385 $(M+H)^+$, 402 $(M+NH_4)^+$
IR (KBr): $\nu$ = 3222, 3084, 1722, 1466, 1412, 1278, 1124, 1084, 1037, 713 $cm^{-1}$

Methode B:

Eine Suspension von 0,92 g (2,00 mmol) des Thionocarbonats in 50 ml wasserfreiem Toluol wurde mit 44 mg (0,27 mmol) $\alpha,\alpha'$-Azoisobutyronitril und 3,90 ml (14,50 mmol) tri-n-Butylzinnhydrid versetzt und 15 min zum Rückfluß erhitzt. Nach dem Abkühlen wurde die Reaktionsmischung im Vakuum zur Trockene eingeengt und der Rückstand an 60 g Kieselgel (Toluol : Ethylacetat 1:1) filtriert. Man erhielt 494 mg (62% der Theorie) eines Gemisches aus 1-(5-O-Benzoyl-2 und 3-desoxy-$\beta$-D-ribofuranosyl)-5-trifluormethyluracil als farbloses Pulver.
$R_f$ = 0,46 (Ethylacetat)
IR (KBr): $\nu$ = 3393, 3062, 1720, 1677, 1482, 1282, 1122, 1056, 710 $cm^{-1}$
Dieses Produkt wurde in 17 ml wasserfreiem Acetonitril gelöst, mit 429 mg (2,40 mmol) 1,1'-Thiocarbonyldiimidazol versetzt und 20 h bei Raumtemperatur gerührt. Das Lösemittel wurde im Vakuum abgedampft und der Rückstand an 44 g Kieselgel (Toluol : Ethylacetat 1:1) chromatographiert. Man erhielt 417 mg (68% der Theorie) des entsprechenden Thiocarbonylimidazolids als farblosen Schaum.
$R_f$ = 0,35 (Ethylacetat)
MS (FAB) m/z = 511 $(M+H)^+$
IR (KBr): $\nu$ = 1700, 1468, 1394, 1338, 1279, 1189, 1115, 1040, 970, 752, 710 $cm^{-1}$
Dieses Produkt wurde in 3 ml wasserfreiem Toluol suspendiert, mit 4 mg (0,02 mmol) $\alpha,\alpha'$-Azoisobutyronitril und 293 µl (1,09 mmol) tri-n-Butylzinnhydrid versetzt und 30 Min zum Rückfluß erhitzt. Nach dem Abkühlen wurde das Lösemittel im Vakuum abgedampft und der Rückstand an 250 g Kieselgel (Toluol : Ethylacetat 1:1) chromatographiert. Man erhielt 262 mg (85% der Theorie) der Titelverbindung als farblosen Schaum. Die physikalischen Daten waren identisch mit denen der nach Methode A erhaltenen Verbindung.

Beispiel 14

2',3'-Didesoxy-5'-O-(4-methylbenzoyl)-5-(trifluormethyl)-uridin

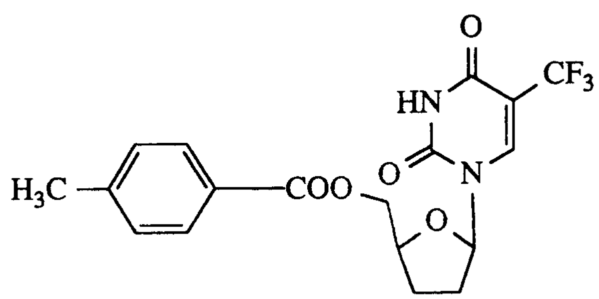

Eine Suspension von 347 mg (0,66 mmol) der Verbindung aus Beispiel VI in 15 ml wasserfreiem Toluol wurde mit 14 mg (0,09 mmol) $\alpha,\alpha'$-Azoisobutyronitril und 1,30 ml (4,80 mmol) tri-n-Butylzinnhydrid versetzt und 30 min zum Rückfluß erhitzt. Nach dem Abkühlen wurde die Reaktionsmischung im Vakuum zur Trockene eingeengt und der Rückstand an 20 g Kieselgel (Toluol : Ethylacetat 3:2) filtriert. Man erhielt 243 mg (70% der Theorie) der Titelverbindung als farblosen Schaum.
$R_f$ = 0,30 (Toluol : Ethylactat 3:2)
IR (KBr): $\nu$ = 3422, 1720, 1466, 1281, 1123, 753, 616 $cm^{-1}$
MS (FAB) m/z = 399 $(M+H)^+$

**Patentansprüche**

**1.** 2',3'-Didesoxy-5-trifluormethyluridine der allgemeinen Formel (I)

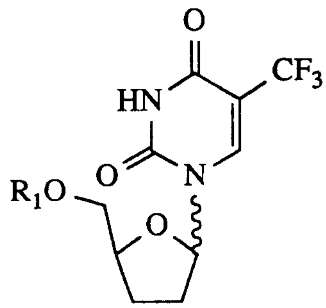

(I),

worin

R[1] für Wasserstoff oder eine Hydroxyschutzgruppe steht,
für 4-Methylbenzoyl oder geradkettiges oder verzweigtes Acyl mit bis zu 18 Kohlenstoffatomen steht, das gegebenenfalls durch Carboxy substituiert ist,
für eine Aminosäure steht, die gegebenenfalls durch eine in der Peptidchemie übliche Aminoschutzgruppe substituiert ist,
für einen in vivo spaltbaren Esterrest steht, oder
für einen Rest der Formeln

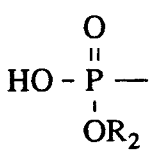

,

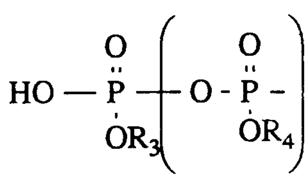

,

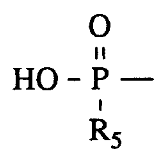

steht, worin

$R^2$, $R^3$ und $R^4$ gleich oder verschieden sind und Wasserstoff oder ein Alkali- oder Erdalkalimetallatom bedeuten,

n die Zahl 1 oder 2 bedeutet,

$R^5$ geradkettiges oder verzweigtes Alkyl mit bis zu 6 Kohlenstoffatomen bedeutet, das gegebenenfalls durch Halogen substituiert ist

und deren physiologisch unbedenklichen Salze.

**2.** Verbindungen der allgemeinen Formel (I) gemäß Anspruch 1,

in welcher

R[1] für Wasserstoff, tert.Butyldimethylsilyl, Trimethylsilyl, Triphenylmethyl, Benzoyl, 4-Methylbenzoyl oder Acetyl steht, oder
für geradkettiges oder verzweigtes Acyl mit bis zu 16 Kohlenstoffatomen steht, das gegebenenfalls durch Carboxy substituiert ist, oder für Leucin (Leu), Valin (Val), Isoleucin (Ile), Phenylalanin (Phe), Asparaginsäure (Asp) oder Glutaminsäure (Glu) steht, die gegebenenfalls an der NH-Funktion durch tert.Butoxycarbonyl (Boc) oder Benzyloxycarbonyl (Z) substituiert sind, oder
für einen in vivo leicht spaltbaren Esterrest der Formeln

$(CH_3)_3C\text{-}CO\text{-}O\text{-}CH_2\text{-}$ ,

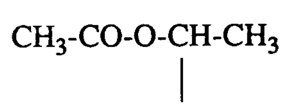

,

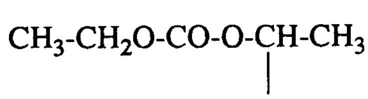

steht, oder

für einen Rest der Formeln

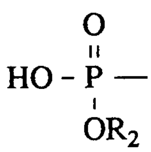 , 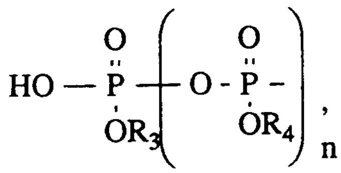 , 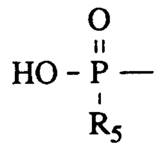

steht, worin

$R^2$, $R^3$ und $R^4$ gleich oder verschieden sind und Wasserstoff oder ein Natriummetallatom bedeuten,

n die Zahl 1 oder 2 bedeutet,

$R^5$ geradkettiges oder verzweigtes Alkyl mit bis zu 4 Kohlenstoffatomen bedeutet, das gegebenenfalls durch Chlor substituiert ist

und deren physiologisch unbedenklichen Salze.

**3.** Verbindungen der allgemeinen Formel (I), gemäß Anspruch 1,

in welcher

$R^1$ für Wasserstoff, tert.Butyldimethylsilyl, 4-Methylbenzoyl oder Benzoyl steht, oder für geradkettiges oder verzweigtes Acyl mit bis zu 14 Kohlenstoffatomen steht, das gegebenenfalls durch Carboxy substituiert ist, oder für Leucin (Leu) oder Asparaginsäure (Asp) steht, die gegebenenfalls an der -NH-Funktion durch die Schutzgruppe Boc substituiert sind, oder für einen in vivo leicht spaltbaren Esterrest der Formeln

$(CH_3)_3C\text{-}CO\text{-}O\text{-}CH_2\text{-}$ , $CH_3\text{-}CO\text{-}O\text{-}CH\text{-}CH_3$ ,

$CH_3\text{-}CH_2O\text{-}CO\text{-}O\text{-}CH\text{-}CH_3$

steht, oder für einen Rest der Formeln

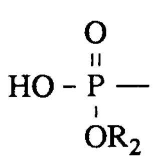 , 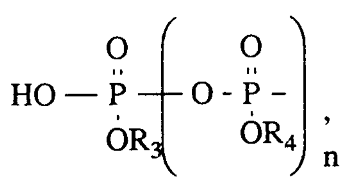 , 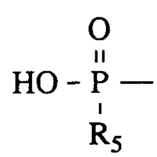

steht, worin

$R^2$, $R^3$ und $R^4$ gleich oder verschieden sind und Wasserstoff oder ein Natriummetallatom bedeuten,

n die Zahl 1 oder 2 bedeutet,

$R^5$ Methyl, Ethyl oder Chlormethyl bedeutet

und deren physiologisch unbedenklichen Salze.

**4.** Arzneimittel enthaltend eine oder mehrere Verbindungen der Ansprüche 1 bis 3.

**5.** Arzneimittel zur Bekämpfung von Hepatitis B Virus enthaltend eine oder mehrere der Verbindungen der

Ansprüche 1 bis 3.

Europäisches Patentamt

# EUROPÄISCHER RECHERCHENBERICHT

Nummer der Anmeldung

EP 92 10 5039
Seite 1

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 217 580 (THE WELLCOME FOUNDATION LIMITED)<br>* Zusammenfassung *<br>* Seite 43, Zeile 23 - Seite 44, Zeile 28; Beispiel 34 * | 1-5 | C07H19/06<br>C07H19/10<br>C07D405/04<br>C07D405/14<br>A61K31/70<br>A61K31/505 |
| Y | EP-A-0 322 384 (MEDIVIR AKTIEBOLAG C/O STATENS BAKTERIOLOGISKA LABORATORIUM)<br>* Zusammenfassung *<br>* Seite 2, Zeile 5 - Zeile 41 *<br>* Anspruch 1 * | 1-5 | |
| Y | EP-A-0 409 575 (THE UNIVERSITY OF BIRMINGHAM)<br>* Anspruch 1 *<br>* Seite 3, Zeile 39 - Zeile 50 * | 1-5 | |
| Y | DE-A-3 048 877 (K.K.GAURI)<br>* Ansprüche 1,4 * | 1-5 | |
| D,Y | EP-A-0 302 760 (THE GOVERNORS OF THE UNIVERSITY OF ALBERTA)<br>* Zusammenfassung * | 1-5 | **RECHERCHIERTE SACHGEBIETE (Int. Cl.5)** |
| D,Y | WO-A-9 014 079 (THE UNITED STATES OF AMERICA)<br>* Zusammenfassung * | 1-5 | C07H<br>C07D<br>A61K |
| Y | CHEMICAL ABSTRACTS, vol. 108, 1988, Columbus, Ohio, US;<br>abstract no. 109067B,<br>K.H.RAND ET AL.: 'Trifluorothymidine: Potential non-invasive Diagnosis of Herpes Simplex Infection Using Fluorine-19 Nuclear Magnetic Resonance in a Murine Hepatitis Model'<br>Seite 319 ;Spalte 2 ;<br>* Zusammenfassung *<br>& J. VIROL. METHODS<br>Bd. 18, Nr. 4, 1987,<br>Seiten 257 - 269; | 1-5 | |

-/--

Der vorliegende Recherchenbericht wurde für alle Patentansprüche erstellt

| Recherchenort | Abschlußdatum der Recherche | Prüfer |
|---|---|---|
| DEN HAAG | 27 APRIL 1992 | SCOTT J.R. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 92 10 5039
Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 206 497 (THE WELLCOME FOUNDATION LIMITED) * Seite 2, Zeile 24 - Seite 3, Zeile 8 * | 1-5 | |
| | ----- | | |
| | | | RECHERCHIERTE SACHGEBIETE (Int. Cl.5) |

Der vorliegende Recherchenbericht wurde für alle Patentansprüche erstellt

| Recherchenort | Abschlußdatum der Recherche | Prüfer |
|---|---|---|
| DEN HAAG | 27 APRIL 1992 | SCOTT J.R. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)